# EUROPÄISCHE PATENTSCHRIFT

(11) **EP 0 772 593 B1**
(45) Veröffentlichungstag und Bekanntmachung des Hinweises auf die Patenterteilung: **02.05.2003**
(21) Anmeldenummer: 95926959.8
(22) Anmeldetag: 19.07.1995
(51) Int. Cl.: C07D 213/70, A61K 31/44, A61K 31/505, C07D 213/80, C07D 401/12, C07D 405/12, C07D 409/12, C07D 417/12, C07D 401/14

(54) **PYRIDYLTHIOVERBINDUNGEN ZUR BEKÄMPFUNG VON HELICOBACTER-BAKTERIEN**
THIOPYRIDYL COMPOUNDS FOR CONTROLLING HELICOBACTER BACTERIA
COMPOSES DE THIOPYRIDYLE UTILES POUR COMBATTRE DES BACTERIES HELICOBACTER

(30) Priorität: 20.07.1994 CH 230394
(43) Veröffentlichungstag der Anmeldung: 14.05.1997
(73) Patentinhaber: ALTANA Pharma AG, 78467 Konstanz (DE)
(72) Erfinder: HANAUER, Guido, D-78465 Konstanz (DE); SIMON, Wolfgang-Alexander, D-78464 Konstanz (DE); ZIMMERMANN, Peter, D-78462 Konstanz (DE); OPFERKUCH, Wolfgang, D-44801 Bochum (DE); KOHL, Bernhard, D-78465 Konstanz (DE); GRUNDLER, Gerhard, D-78464 Konstanz (DE); SENN-BILFINGER, Jörg, D-78464 Konstanz (DE)
(86) Internationale Anmeldenummer: EP9502851
(87) Internationale Veröffentlichungsnummer: WO96002505

(56) Entgegenhaltungen:
- WO-A-94/13290
- CHEMICAL ABSTRACTS, vol. 108, no. 21, 23. Mai 1988, Columbus, Ohio, US; abstract no. 186590u, M. OTA ET AL. 'Preparation of 2-pyridylmethylthio- or 2-pyridylmethylsulfinyl-substituted cyclic compounds for preventing or treating gastropathy.' Seite 691 ;Spalte 2 ; & JP,A,62 209 062 (YAMANOUCHI PHARMACEUTICAL CO., LTD.) 14. September 1987

## Beschreibung

### Anwendungsgebiet der Erfindung

Die Erfindung betrifft Verbindungen, die in der pharmazeutischen Industrie als Wirkstoffe für die Herstellung von Arzneimitteln verwendet werden sollen.

### Bekannter technischer Hintergrund

Aus der Internationalen Patentanmeldung WO-A-94/13290 sind gegen Helicobacter-Bakterien wirksame 2-Pyridinylmethylthio-Verbindungen bekannt, die am Schwefel einen Benzimidazol-2-ylrest trage. In den Chemical Abstracts **108**: 186590u werden 2-Pyridinylmethylthio-Verbindungen beschrieben, die sich zur Behandlung von Magenerkrankungen eignen sollen, die am Schwefel einen Chinolin- bzw. Chinazolinrest tragen.

### Beschreibung der Erfindung

Gegenstand der Erfindung sind Verbindungen der Formel I (siehe beigefügtes Formelblatt), worin
- R: Wasserstoff, 1-4C-Alkyl, Halogen, Trifluormethyl, 1-4C-Alkoxycarbonyl, Carboxy oder Cyan bedeutet,
- R1: Wasserstoff oder 1-4C-Alkyl bedeutet,
- R2: Wasserstoff oder 1-4C-Alkyl bedeutet,
- R3: Wasserstoff, 1-4C-Alkyl, 1-4C-Alkoxy oder Halogen bedeutet,
- R4: einen Mono- oder Di-1-4C-alkylcarbamoyl- oder -thiocarbamoylrest, einen N-1-4C-Alkyl-N'-cyan-amidinorest, einen 1-N-1-4C-Alkylamino-2-nitroethylenrest, einen N-2-Propinyl-N'-cyan-amidinorest, einen Aminosulfonyl-amidinorest, den Rest -N(R7)R8 oder einen durch R9 und R10 substituierten Cyclus oder Bicyclus bedeutet, der ausgewählt ist aus der Gruppe bestehend aus Benzol, Naphthalin, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Thiadiazol-1-oxid, Oxadiazol, Pyridin, Pyridin-N-oxid, Pyrimidin, Triazin, Pyridon, Benzimidazol, Imidazopyridin, Benzthiazol, Benzoxazol und Chinolin,
- R5: Wasserstoff, 1-4C-Alkyl, 1-4C-Alkoxy oder Halogen bedeutet,
- R6: Wasserstoff oder 1-4C-Alkyl bedeutet,
- R7: 1-7C-Alkyl, 3-7C-Cycloalkyl oder Ar-1-4C-alkyl und
- R8: 1-7C-Alkyl, 3-7C-Cycloalkyl oder Ar-1-4C-alkyl bedeutet,
wobei
- Ar: Phenyl, Furyl, Naphthyl, Tetrahydronaphthyl oder durch R11, R12 und R13 substituiertes Phenyl bedeutet,
oder worin
- R7 und R8: gemeinsam und unter Einschluß des Stickstoffatoms, an das beide gebunden sind, einen unsubstituierten oder substituierten 5- oder 6-Ring-Hetero(bi)cyclus darstellen, der ausgewählt ist aus der Gruppe bestehend aus Piperidin, Piperazin, Morpholin, Indolin, 1,2,3,4-Tetrahydrochinolin und 1,2,3,4-Tetrahydroisochinolin,
wobei
- ein substituierter Piperidinorest substituiert ist mit einem, zwei oder drei gleichen oder verschiedenen Substituenten ausgewählt aus der Gruppe bestehend aus 1-4C-Alkyl, 1-4C-Alkoxycarbonyl, Hydroxy-1-4C-alkyl, Phenyl, durch R11, R12 und R13 substituiertes Phenyl, Phenyl-1-4C-alkyl, Benzoyl, durch Halogen substituiertes Benzoyl und Carboxy,
- ein substituierter Piperazinorest in 2-, 3-, 5- oder 6-Position substituiert sein kann mit einem 1-4C-Alkylrest und in 4-Position substituiert ist mit einem Substituenten ausgewählt aus der Gruppe bestehend aus 1-4C-Alkyl, 3-7C-Cycloalkyl, 3-7C-Cycloalkyl-1-4C-alkyl, 1-4C-Alkoxycarbonyl-1-4C-alkyl, Carbamoyl, -CₚH₍₂ₚ₋₂₎-R14 und -C_{q}H_{2q}-R14,
- ein substituierter Morpholinorest substituiert ist mit einem oder zwei gleichen oder verschiedenen 1-4C-Alkylresten,
- ein substituierter Indotin-1-ylrest in 2- und/oder 3-Position substituiert sein kann durch eine Carboxygruppe oder durch einen oder zwei gleiche oder verschiedene 1-4C-Alkylreste, und im Benzoteil substituiert sein kann mit einem oder zwei gleichen oder verschiedenen Substituenten ausgewählt aus der Gruppe bestehend aus 1-4C-Alkyl, Halogen und Nitro,
- ein substituierter 1,2,3,4-Tetrahydrochinolinrest substituiert ist mit einem oder zwei gleichen oder verschiedenen Substituenten ausgewählt aus der Gruppe bestehend aus 1-4C-Alkyl und Halogen,
- ein substituierter 1,2,3,4-Tetrahydroisochinolinrest substituiert ist mit einem oder zwei gleichen oder verschiedenen Substituenten ausgewählt aus der Gruppe bestehend aus 1-4C-Alkyl, Carboxy und Phenyl,
- R9: Wasserstoff, 1-4C-Alkyl, Hydroxy, 1-4C-Alkoxy, Halogen, Nitro, Guanidino, Carboxy, 1-4C-Alkoxycarbonyl, durch R15 substituiertes 1-4C-Alkyl oder -N(R16)R17 bedeutet,
- R10: Wasserstoff, 1-4C-Alkyl, Hydroxy, 1-4C-Alkoxy, Halogen oder Trifluormethyl bedeutet,
- R11: Wasserstoff, 1-4C-Alkyl, Hydroxy, 1-4C-Alkoxy, 1-4C-Alkylcarbonyl, Halogen, 1-4C-Alkylamino oder Nitro,
- R12: Wasserstoff, 1-4C-Alkyl, Hydroxy, 1-4C-Alkoxy, Halogen oder Nitro, und
- R13: Wasserstoff oder Trifluormethyl bedeutet,
- R14: einen durch R9 und R10 substituierten Cyclus oder Bicyclus bedeutet, der ausgewählt ist aus der Gruppe bestehend aus Benzol, Naphthalin, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Oxadiazol, Pyridin, Pyridin-N-oxid, Pyrimidin, Benzimidazol und Chinolin,
- R15: Hydroxy, 1-4C-Alkoxy, Carboxy, 1-4C-Alkoxycarbonyl oder -N(R16)R17 bedeutet, wobei
- R16: Wasserstoff, 1-4C-Alkyl oder -CO-R18 und
- R17: Wasserstoff oder 1-4C-Alkyl bedeutet, oder wobei
- R16 und R17: zusammen und unter Einschluß des Stickstoffatoms, an das beide gebunden sind, einen Piperidino- oder Morpholinorest darstellen,
- R18: Wasserstoff, 1-4C-Alkyl oder 1-4C-Alkoxy bedeutet,
- W: CH oder N bedeutet,
- X: 0 (Sauerstoff), N-1-4C-Alkyl oder S bedeutet,
- Y: 0 (Sauerstoff), N-1-4C-Alkyl, S, SO oder SO₂ bedeutet,
- Z: 0 (Sauerstoff), N-1-4C-Alkyl, S, SO oder SO₂ bedeutet,
- m: eine Zahl von 1 bis 7 bedeutet,
- n: die Zahl 0, 1 oder 2 bedeutet,
- r: eine Zahl von 2 bis 4 bedeutet,
- t: die Zahl 0 oder 1 bedeutet,
- u: eine Zahl von 0 bis 4 bedeutet,
- v: die Zahl 0 oder 1 bedeutet,
- p: eine Zahl von 2 bis 4 bedeutet und
- q: eine Zahl von 0 bis 4 bedeutet
und ihre Salze,
wobei
- t: und/oder v nicht die Zahl 1 bedeuten, wenn m die Zahl 1 bedeutet,
- Z: nicht die Bedeutung SO oder SO₂ hat, wenn u die Zahl 0 bedeutet, und wobei
- R4: nicht -N(R7)R8 oder einen über N (Stickstoff) gebundenen Cyclus oder Bicyclus bedeutet, wenn Z O, S, SO oder SO₂, v die Zahl 1 und u die Zahl 0 bedeutet.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

Halogen im Sinne der vorliegenden Erfindung ist Brom, Chlor und Fluor.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

1-4C-Alkoxycarbonyl steht für einen Rest, der neben der Carbonylgruppe einen der vorstehend genannten 1-4C-Alkoxyreste enthält. Beispielsweise seien der Methoxycarbonyl- und der Ethoxycarbonylrest genannt.

Mono- oder Di-1-4C-alkylcarbamoylreste sind Carbamoylreste (-CO-NH₂), die durch einen oder zwei gleiche oder verschiedene der vorstehend genannten 1-4C-Alkylreste substituiert sind. Beispielsweise sei der Methylcarbamoyl-, der Isopropylcarbamoyl- und der Dimethylcarbamoylrest genannt.

Mono- oder Di-1-4C-alkylthiocarbamoylreste sind Thiocarbamoylreste (-CS-NH₂), die durch einen oder zwei gleiche oder verschiedene der vorstehend genannten 1-4C-Alkylreste substituiert sind. Beispielsweise sei der Methylthiocarbamoyl-, der Isopropylthiocarbamoyl- und der Dimethylthiocarbamoylrest genannt.

Als N-1-4C-Alkyl-N'-cyan-amidinorest sei beispielsweise insbesondere der N-Methyl-N'-cyan-amidinorest [-C(=NCN)-NH-CH₃] genannt.

Als 1-N-1-4C-Alkylamino-2-nitroethylenrest sei beispielsweise insbesondere der 1-N-Methylamino-2-nitroethylenrest [-C(NHCH₃)=CHNO₂] genannt, wobei die Reste -NHCH₃ und -NO₂ cis- oder transständig zueinander sein können.

1-7C-Alkyl steht für geradkettige oder verzweigte Alkylreste mit 1 bis 7 Kohlenstoffatomen. Beispielsweise seien genannt der Heptyl-, Isoheptyl-(2-Methylhexyl-), Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkyl steht für Cycloalkylreste mit 3 bis 7 Kohlenstoffatomen, also für den Cyclopropyl-, den Cyclobutyl-, den Cyclopentyl-, den Cyclohexyl- und den Cycloheptylrest.

Ar-1-4C-alkyl steht für einen der obengenannten, durch Ar substituierten 1-4C-Alkylrest. Beispielsweise seien der Phenethyl-, der Benzyl-, der 2-Furylmethyl- (= Furfuryl-) und der 1-Naphthylmethylrest genannt.

Hydroxy-1-4C-alkyl steht für einen der vorstehend genannten 1-4C-Alkylreste, der durch Hydroxy substituiert ist. Beispielsweise seien der Hydroxymethylrest, der 2-Hydroxyethylrest oder der 3-Hydroxypropylrest genannt.

3-7C-Cycloalkyl-1-4C-alkyl steht für einen der vorstehend genannten 1-4C-Alkylreste, der durch einen der vorstehend genannten 3-7C-Cycloalkylreste substituiert ist. Beispielsweise seien der Cyclopropylmethyl-, der Cyclohexylmethyl- und der Cyclohexylethylrest genannt.

1-4C-Alkoxycarbonyl-1-4C-alkyl steht für einen der vorstehend genannten 1-4C-Alkylreste, der durch einen der vorstehend genannten 1-4C-Alkoxycarbonylreste substituiert ist. Beispielsweise sei der Ethoxycarbonylmethylrest genannt.

Als beispielhafte, durch R15 substituierte 1-4C-Alkylreste seien der 2-Methoxycarbonylethyl-, der 2-Ethoxycarbonylethyl-, der Methoxycarbonylmethyl-, der Carboxymethyl-, der 2-Hydroxyethyl-, der Methoxymethyl-, der 2-Methoxyethyl-, der Dimethylaminomethyl- und der 2-Dimethylaminoethylrest genannt.

1-4C-Alkylcarbonyl steht für einen Rest, der neben der Carbonylgruppe einen der vorstehend genannten 1-4C-Alkylreste enthält. Beispielsweise sei der Acetylrest genannt.

Als Reste -CₘH₂ₘ-, -CᵣH₂ᵣ-, -CᵤH₂ᵤ- und -C_{q}H_{2q}- kommen geradkettige oder verzweigte Reste infrage. Beispielsweise seien genannt der Heptylen-, Isoheptylen- (2-Methylhexylen-), Hexylen-, Isohexylen- (2-Methylpentylen-), Neohexylen- (2,2-Dimethylbutylen-), Pentylen-, Isopentylen- (3-Methylbutylen-), Neopentylen- (2,2-Dimethylpropylen-), Butylen-, iso-Butylen-, sec.-Butylen-, tert.-Butylen-, Propylen-, Isopropylen-, Ethylen- und der Methylenrest.

Als Reste -CₘH₂ₘ- sind bevorzugt der Ethylen- (-CH₂CH₂-), der Butylen-(-CH₂CH₂CH₂CH₂-) und insbesondere der Propylenrest (-CH₂CH₂CH₂-) zu nennen.

Als Rest -CᵣH₂ᵣ- ist bevorzugt der Methylenrest zu nennen bzw. in einer weiteren bevorzugten Ausführungsform stellt r die Zahl 0 dar, so daß der Ausdruck CᵣH₂ᵣ verschwindet bzw. einen Bindungsstrich darstellt.

In einer weiteren bevorzugten Ausführungsform stellt u die Zahl 0 dar, so daß der Ausdruck CᵤH₂ᵤ verschwindet bzw. einen Bindungsstrich darstellt und der Rest R4 direkt an die Gruppe Z gebunden ist.

In einer Ausführungsform steht t für die Zahl 1.

In einer weiteren Ausführungsform steht t für die Zahl 0, so daß der Ausdruck Y-CᵣH₂ᵣ verschwindet bzw. einen Bindungsstrich darstellt.

In einer weiteren bevorzugten Ausführungsform steht v für die Zahl 0, so daß der Ausdruck Z-CᵤH₂ᵤ verschwindet bzw. einen Bindungsstrich darstellt.

Als Reste -CₚH₍₂ₚ₋₂₎- sind der Vinylen-, der 2-Butenylen-, der 3-Butenylenund insbesondere der 1-Propenylen- und der 2-Propenylenrest zu nennen.

Als Rest -C_{q}H_{2q}- ist bevorzugt der Methylenrest zu nennen bzw. in einer weiteren bevorzugten Ausführungsform stellt q die Zahl 0 dar, so daß der Ausdruck C_{q}H_{2q} verschwindet bzw, einen Bindungsstrich darstellt.

Die Substituenten R9 und R10 können in den Cyclen bzw. Bicyclen R4 an jeder denkbaren Position angebunden sein. Als beispielhafte, durch R9 und R10 substituierte Reste R4 seien genannt: 4-Methylphenyl, 3-Dimethylaminomethylphenyl, 3-Piperidinomethylphenyl, 3-Carboxymethylphenyl, 2-Dimethylaminomethyl-5-methyl-3-furyl, 1-Methylpyrrol-3-yl, 4,5-Dimethyl-oxazol-2-yl 3,5-Dimethyl-isoxazol-4-yl, 4,5-Dimethyl-thiazol-2-yl, 4-Methyl-5-carboxymethyl-thlazol-2-yl, 1-Methyl-imidazol-2-yl, 1-Methyl-pyrazol-3-yl, 1-(2-Dimethylaminoethyl)-pyrazol-3-yl, 5-Methyl-1,3,4-oxadiazol-2-yl, 1-Methyl-1,2,3-triazol-4-yl, 1-Methyl-1,2,4-trlazol-3-yl, 1-(2-Dimethylaminoethyl)-1,2,3-triazol-4-yl, 1-Methyl-tetrazol-5-yl, 1-(2-Dimethylaminoethyl)-tetrazol-5-yl, 1-Carboxymethyl-tetrazol-5-yl, 5-Methyl-1,3,4-thiadiazol-2-yl, 5-Trifluormethyl-1,3,4-thiadiazol-2-yl, 1-(2-Hydroxyethyl)-tetrazol-5-yl, 2-Amino-1,3,4-thiadiazol-2-yl, 3-Amino-1,2,4-triazol-5-yl, 4-Methyl-5-trifluormethyl-1,2,4-triazol-3-yl, 4-Amino-pyrimidin-2-yl, 3-Methyl-2-furyl, 2-Methyl-3-furyl, 5-Methyl-2-furyl, 5-Ethyl-2-furyl, 3-Methoxy-2-furyl, 5-Dimethylaminomethyl-2-furyl, 5-N-Morpholinomethyl-2-furyl, 5-Methoxymethyl-2-furyl, 5-Hydroxymethyl-2-furyl, 5-N-Piperidinomethyl-2-furyl, 5-Chlor-2-furyl, 5-Fluor-2-furyl, 5-Methyl-2-thienyl, 5-Chlor-2-thienyl, 3-Methyl-2-thienyl, 3-Amino-2-thienyl, 3-Guanidino-2-thienyl, 3-Methoxy-2-thienyl, 2-Methyl-3-thienyl, 5-Dimethylaminomethyl-2-thienyl, 5-N-Morpholinomethyl-2-thienyl, 5-Methyl-2-pyrrolyl, 2,5-Dimethyl-1-pyrrolyl, 1,5-Dimethyl-2-pyrrolyl, 1-Methyl-2-pyrrolyl, 2-Amino-4-thiazolyl, 2-Methyl-4-thiazolyl, 2-Amino-5-methyl-4-thiazolyl, 4-Methyl-5-thiazolyl, 2-Dimethylaminomethyl-4-thiazolyl, 2-Guanidino-4-thiazolyl, 2-Formylamino-4-thiazolyl, 2-N-Morpholinomethyl-4-thiazolyl, 4-Methyl-5-oxazolyl, 3-Guanidino-1-pyrazolyl, 3-Guanidino-4-pyrazolyl, 2-Methyl-4-imidazolyl, 5-Methyl-4-imidazolyl, 2-Methyl-1-imidazolyl, 2-Methyl-5-nitro-1-imidazolyl, 4,5-Dimethyl-2-imidazolyl, 4-Hydroxymethyl-5-methyl-1-imidazolyl, 3-Methyl-1-pyrazolyl, 5-Amino-1,2,4-thiadiazol-3-yl, 4-Methoxy-2-pyridinyl, 4-Methoxy-3-methyl-2-pyridinyl und 3,4-Dimethoxypyridinyl.

Als beispielhafte, durch R11, R12 und R13 substituierte Phenylreste seien die Reste 3,4-Dihydroxy-, 3-Hydroxy-4-methoxy-, 3,4-Dimethoxy-, 2-Methoxy-, 2-Ethoxy-, 3-Methoxy-, 4-Methoxy-, 2-Hydroxy-, 3-Hydroxy-, 4-Hydroxy-, 3,4-Dihydroxy-, 4-Acetyl-, 4-Fluor-, 4-Chlor-, 2-Chlor-, 3-Chlor-, 3,4-Dichlor-, 3-Trifluormethyl-, 2-Trifluormethyl-, 2-Methyl-, 3-Methyl-, 4-Methyl-, 2,3-Dimethyl-, 2,4-Dimethyl-, 3,4-Dimethyl-, 2,5-Dimethyl-, 4-Nitro-, 2,6-Dinitro-4-trifluormethyl- und 5-Chlor-2-methylaminophenyl genannt.

Als substituierte Piperidinoreste seien beispielsweise der 2-Carboxypiperidino-, 2-n-Propylpiperidino-, 5-Ethyl-2-methylpiperidino-, 4-Hydroxymethyl-4-phenylpiperidino-, 4-n-Propylpiperidino-, 4-(3-Phenylpropyl)piperidino-, 2,6-Dimethylpiperidino-, 4-Phenyl-4-propyloxycarbonylpiperidino, 4-Ethoxycarbonyl-4-phenylpiperidino-, 4-Carboxy-4-phenylpiperidino-, 4-Carboxypiperidino-, 4-(4-Fluorbenzoyl)-piperidino-, 4-(4-Chlorbenzoyl)-piperidino-, 2,3-Dicarboxypiperidino-, 2,4-Dicarboxypiperidino-, 2,6-Dicarboxypiperidino-, 2-Ethoxycarbonylpiperidino-, 2-Methylpiperidino-, 2,6-Dimethylpiperidino-, 2-Hydroxymethylpiperidino-, 2-Ethylpiperidino-, 2-(2-Hydroxyethyl)-piperidino-, 3-Ethoxycarbonylpiperidino- und der 4-Benzylpiperidinorest genannt.

Als substituierte Piperazinoreste seien beispielsweise der 4-Methylpiperiazino-, 4-Phenylpiperazino-, 4-(2-Methylphenyl)piperazino-, 4-(2,3-Dimethylphenyl)piperazino-, 4-(2-Chlorphenyl)piperazino-, 4-(2-Methoxyphenyl)-piperazino-, 4-(2-Ethoxyphenyl)piperazino-, 4-(3-Chlorphenyl)piperazino-, 4-(4-Fluorphenyl)piperazino-, 4-(4-Chlorphenyl)piperazino-, 4-(4-Methoxyphenyl)piperazino-, 4-Carbamoylpiperazino-, 3-Methyl-4-(4-chlorphenyl)piperazino-, 3-Methyl-4-(4-methoxyphenyl)piperazino-, 3-Methyl-4-(4-methylphenyl)piperazino-, 4-(2,4-Dimethylphenyl)piperazino-, 4-(3,4-Dichlorphenyl)piperazino-, 4-(3,4-Dimethylphenyl)piperazino-, 3-Methyl-4-phenylpiperazino-, 3-Methyl-4-(3-chlorphenyl)piperazino-, 4-Benzylpiperazino-, 4-Propylpiperazino-, 4-(3-Methylphenyl)piperazino-, 4-(3-Methoxyphenyl)-piperazino-, 4-(4-Methylphenyl)piperazino-, 4-(2,5-Dimethylphenyl)piperazino-, 4-Cyclopropylpiperazino-, 4-Cyclobutylpiperazino-, 4-Cyclopentylpiperazino-, 4-Cyclohexylpiperazino-, 4-Cycloheptylpiperazino-, 4-n-Butylpiperazino-, 4-iso-Butylpiperazino-, 4-tert.-Butylpiperazino-, 4-(1-Phenylethyl)piperazino-, 4-Ethoxycarbonylmethylpiperazino-, 4-(2-Phenylethyl)-piperazino-, 4-(2-Cyclohexylethyl)piperazino-, 4-(2-Hydroxyphenyl)piperazino-, 4-(3,4-Dimethoxyphenyl)piperazino-, 4-Isopropylpiperazino-, 3-Methyl-4-(3-methoxyphenyl)piperazino-, 4-(4-Hydroxyphenyl)piperazino-, 3-Methyl-4-(3-methylphenyl)piperazino-, 4-(3-Hydroxyphenyl)piperazino-, 4-(2,6-Dinitro-4-trifluormethylphenyl)piperazino-, 4-(4-Nitrophenyl)piperazino-, 4-(4-Acetylphenyl)piperazino-, 4-(2-Chlor-5-thienyl-methyl)piperazino- und der 4-[2-(2-Methyl-5-nitro-1-imidazolyl)ethyl]piperazinorest genannt.

Als substituierter Morpholinorest sei beispielsweise der 3,5-Dimethylmorpholinorest genannt.

Als substituierte Indolin-1-yl-reste seien beispielsweise der 2-Carboxy-1-indolinyl-, 6-Fluor-1-indolinyl-, 5-Brom-1-indolinyl-, 2,7-Dimethyl-1-indolinyl-, 2-Methyl-1-indolinyl-, 5-Brom-7-nitro-1-indolinyl-, 5-Nitro-1-indolinyl-, 2,3-Dimethyl-1-indolinyl- und der 6-Nitro-1-indolinylrest genannt.

Als substituierte 1,2,3,4-Tetrahydrochinolinreste seien beispielsweise der 2-Ethoxycarbonyl-1,2,3,4-tetrahydro-1-chinolinyl-, 2-Methyl-1,2,3,4-tetrahydro-1-chinolinyl-, 6-Methyl-1,2,3,4-tetrahydro-1-chinolinyl-, 6-Fluor-2-methyl-1,2,3,4-tetrahydro-1-chinolinyl-, 4-Methyl-1,2,3,4-tetrahydro-1-chinolinyl- und der 2-Fluor-6-methyl-1,2,3,4-tetrahydro-1-chinolinylrest genannt.

Als substituierter 1,2,3,4-Tetrahydroisochinolinrest sei beispielsweise der 3-Carboxy-1,2,3,4-tetrahydro-2-isochinolinylrest genannt.

Als Salze kommen für Verbindungen der Formel I, in denen n die Zahl 0 bedeutet, alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Für Verbindungen der Formel I, in denen n die Zahlen 1 oder 2 bedeutet, und/oder für Verbindungen mit Carboxygruppe kommen als Salze auch Salze mit Basen in Betracht. Als Beispiele für basische Salze seien Lithium-, Natrium-, Kalium-, Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Hervorzuhebende Verbindungen sind solche der Formel I, worin
- R: Wasserstoff, 1-4C-Alkyl oder Halogen bedeutet,
- R1: Wasserstoff bedeutet,
- R2: Wasserstoff oder 1-4C-Alkyl bedeutet,
- R3: Wasserstoff, 1-4C-Alkyl, 1-4C-Alkoxy oder Halogen bedeutet,
- R4: einen Mono- oder Di-1-4C-alkylthiocarbamoylrest, den Rest -N(R7)R8 oder einen durch R9 und R10 substituierten Cyclus oder Bicyclus bedeutet, der ausgewählt ist aus der Gruppe bestehend aus Benzol, Naphthalin, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Thiadiazol-1-oxid, Oxadiazol, Pyridin, Pyridin-N-oxid, Pyrimidin, Triazin, Pyridon, Benzimidazol, Imidazopyridin, Benzthiazol, Benzoxazol und Chinolin,
- R5: Wasserstoff oder 1-4C-Alkyl bedeutet,
- R6: Wasserstoff oder 1-4C-Alkyl bedeutet,
- R7: 1-7C-Alkyl und
- R8: Ar-1-4C-alkyl bedeutet,
wobei
- Ar: Phenyl, Furyl, Naphthyl, Tetrahydronaphthyl oder durch R11, R12 und R13 substituiertes Phenyl bedeutet,
oder worin
- R7 und R8: gemeinsam und unter Einschluß des Stickstoffatoms, an das beide gebunden sind, einen unsubstituierten oder substituierten 5- oder 6-Ring-Hetero(bi)cyclus darstellen, der ausgewählt ist aus der Gruppe bestehend aus Piperidin, Piperazin, 1,2,3,4-Tetrahydrochinolin und 1,2,3,4-Tetrahydroisochinolin,
wobei
- ein substituierter Piperidinorest substituiert ist mit einem, zwei oder drei gleichen oder verschiedenen Substituenten ausgewählt aus der Gruppe bestehend aus 1-4C-Alkyl, Phenyl, durch R11, R12 und R13 substituiertes Phenyl und Phenyl-1-4C-alkyl,
- ein substituierter Piperazinorest in 4-Position substituiert ist mit einem Substituenten ausgewählt aus der Gruppe bestehend aus 1-4C-Alkyl, 1-4C-Alkoxycarbonyl-1-4C-alkyl, -CₚH₍₂ₚ₋₂₎-R14 und -C_{q}H_{2q}-R14,
   ein substituierter 1,2,3,4-Tetrahydrochinolinrest substituiert ist mit einem oder zwei gleichen oder verschiedenen Substituenten ausgewählt aus der Gruppe bestehend aus 1-4C-Alkyl und Halogen,
- ein substituierter 1,2,3,4-Tetrahydroisochinolinrest substituiert ist mit einem oder zwei gleichen oder verschiedenen Substituenten ausgewählt aus der Gruppe bestehend aus 1-4C-Alkyl, Carboxy und Phenyl,
- R9: Wasserstoff, 1-4C-Alkyl, Halogen, Nitro, Carboxy, 1-4C-Alkoxycarbonyl oder durch R15 substituiertes 1-4C-Alkyl bedeutet,
- R10: Wasserstoff oder 1-4C-Alkyl bedeutet,
- R11: Wasserstoff, 1-4C-Alkyl, Hydroxy, 1-4C-Alkoxy, Halogen oder Nitro,
- R12: Wasserstoff, 1-4C-Alkyl, Hydroxy oder 1-4C-Alkoxy und
- R13: Wasserstoff bedeutet,
- R14: einen durch R9 und R10 substituierten Cyclus oder Bicyclus bedeutet, der ausgewählt ist aus der Gruppe bestehend aus Benzol, Naphthalin, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Oxadiazol, Pyridin, Pyridin-N-oxid, Pyrimidin, Benzimidazol und Chinolin,
- R15: Carboxy, 1-4C-Alkoxycarbonyl oder -N(R16)R17 bedeutet, wobei
- R16: Wasserstoff oder 1-4C-Alkyl und
- R17: 1-4C-Alkyl bedeutet, oder wobei
- R16 und R17: zusammen und unter Einschluß des Stickstoffatoms, an das beide gebunden sind, einen Piperidino- oder Morpholinorest darstellen,
- W: CH oder N bedeutet,
- X: 0 (Sauerstoff), N-1-4C-Alkyl oder S bedeutet,
- Z: 0 (Sauerstoff), N-1-4C-Alkyl, S oder SO₂ bedeutet,
- m: eine Zahl von 1 bis 6 bedeutet,
- n: die Zahl 0 bedeutet,
- t: die Zahl 0 bedeutet,
- u: eine Zahl von 0 bis 4 bedeutet,
- v: die Zahl 0 oder 1 bedeutet,
- p: eine Zahl von 2 bis 4 bedeutet und
- q: eine Zahl von 0 bis 2 bedeutet
und ihre Salze,
wobei
- v: nicht die Zahl 1 bedeutet, wenn m die Zahl 1 bedeutet,
- Z: nicht die Bedeutung SO₂ hat, wenn u die Zahl 0 bedeutet,
und wobei
- R4: nicht -N(R7)R8 oder einen über N (Stickstoff) gebundenen Cyclus oder Bicyclus bedeutet, wenn Z 0, S oder SO₂ bedeutet, v die Zahl 1 bedeutet und u die Zahl 0 bedeutet.

Besonders hervorzuhebende Verbindungen sind solche der Formel I, worin
- R: Wasserstoff oder 1-4C-Alkyl bedeutet,
- R1: Wasserstoff bedeutet,
- R2: Wasserstoff bedeutet,
- R3: Wasserstoff, 1-4C-Alkyl, 1-4C-Alkoxy oder Halogen bedeutet,
- R4: einen Mono- oder Di-1-4C-alkylthiocarbamoylrest, den Rest -N(R7)R8 oder einen durch R9 und R10 substituierten Cyclus oder Bicyclus bedeutet, der ausgewählt ist aus der Gruppe bestehend aus Benzol, Furan, Thiophen, Thiazol, Isothiazol, Imidazol, Triazol, Tetrazol, Thiadiazol, Pyridin, Pyrimidin, Benzimidazol und Chinolin,
- R5: Wasserstoff bedeutet,
- R6: Wasserstoff oder 1-4C-Alkyl bedeutet,
- R7: 1-7C-Alkyl und
- R8: Ar-1-4C-alkyl bedeutet,
wobei
- Ar: Phenyl bedeutet,
oder worin
- R7 und R8: gemeinsam und unter Einschluß des Stickstoffatoms, an das beide gebunden sind, einen unsubstituierten oder substituierten 5- oder 6-Ring-Hetero(bi)cyclus darstellen, der ausgewählt ist aus der Gruppe bestehend aus Piperidin, Piperazin und 1,2,3,4-Tetrahydroisochinolin,
wobei
- ein substituierter Piperidinorest substituiert ist mit einem, zwei oder drei gleichen oder verschiedenen Substituenten ausgewählt aus der Gruppe bestehend aus 1-4C-Alkyl, Phenyl und Phenyl-1-4C-alkyl,
- ein substituierter Piperazinorest in 4-Position substituiert ist mit einem Substituenten ausgewählt aus der Gruppe bestehend aus 1-4C-Alkyl, 1-4C-Alkoxycarbonyl-]-4C-alkyl, -CₚH₍₂ₚ₋₂₎-R14 und -C_{q}H_{2q}-R14,
- ein substituierter 1,2,3,4-Tetrahydroisochinolinrest substituiert ist mit einem oder zwei gleichen oder verschiedenen Substituenten ausgewählt aus der Gruppe bestehend aus 1-4C-Alkyl und Carboxy,
- R9: Wasserstoff, 1-4C-Alkyl, Halogen, Nitro, Carboxy, 1-4C-Alkoxycarbonyl oder durch R15 substituiertes 1-4C-Alkyl bedeutet,
- R10: Wasserstoff oder 1-4C-Alkyl bedeutet,
- R14: einen durch R9 und R10 substituierten Cyclus oder Bicyclus bedeutet, der ausgewählt ist aus der Gruppe bestehend aus Benzol, Furan, Thiophen, Thiazol, Isothiazol, Imidazol, Triazol, Tetrazol, Thiadiazol, Pyridin, Pyrimidin, Benzimidazol und Chinolin,
- R15: Carboxy, 1-4C-Alkoxycarbonyl oder -N(R16)R17 bedeutet, wobei
- R16: 1-4C-Alkyl und
- R17: 1-4C-Alkyl bedeutet, oder wobei
- R16 und R17: zusammen und unter Einschluß des Stickstoffatoms, an das beide gebunden sind, einen Piperidino- oder Morpholinorest darstellen,
- W: CH oder N bedeutet,
- X: S bedeutet,
- Z: S bedeutet,
- m: eine Zahl von 1 bis 5 bedeutet,
- n: die Zahl 0 bedeutet,
- t: die Zahl 0 bedeutet,
- u: eine Zahl von 0 bis 2 bedeutet,
- v: die Zahl 0 oder 1 bedeutet,
- p: eine Zahl von 2 bis 4 bedeutet und
- q: eine Zahl von 0 bis 2 bedeutet
und ihre Salze,
wobei
- v: nicht die Zahl 1 bedeutet, wenn m die Zahl 1 bedeutet,
und wobei
- R4: nicht -N(R7)R8 oder einen über N (Stickstoff) gebundenen Cyclus oder Bicyclus bedeutet, wenn Z S bedeutet, v die Zahl 1 bedeutet und u die Zahl 0 bedeutet.

Beispielhafte Verbindungen sind solche der Formel I, worin
- R: Wasserstoff bedeutet,
- R1: Wasserstoff bedeutet,
- R2: Wasserstoff bedeutet,
- R3: Wasserstoff, 1-4C-Alkyl oder 1-4C-Alkoxy bedeutet,
- R4: einen Di-1-4C-alkylthiocarbamoylrest, den Rest -N(R7)R8 oder einen durch R9 und R10 substituierten Cyclus oder Bicyclus bedeutet, der ausgewählt ist aus der Gruppe bestehend aus Benzol, Furan, Thiophen, Thiazol, Imidazol, Tetrazol, Pyridin und Benzimidazol,
- R5: Wasserstoff bedeutet,
- R6: Wasserstoff oder 1-4C-Alkyl bedeutet,
- R7 und R8: gemeinsam und unter Einschluß des Stickstoffatoms, an das beide gebunden sind, einen unsubstituierten oder substituierten Piperazinorest oder einen 1,2,3,4-Tetrahydroisochinolinrest bedeuten,
wobei
- ein substituierter Piperazinorest substituiert ist mit einem Substituenten ausgewählt aus der Gruppe bestehend aus -CₚH₍₂ₚ₋₂₎-R14 und -C_{q}H_{2q}-R14,
- R9: Wasserstoff, 1-4C-Alkyl, Halogen, Nitro, Carboxy, 1-4C-Alkoxycarbonyl oder durch R15 substituiertes 1-4C-Alkyl bedeutet,
- R10: Wasserstoff oder 1-4C-Alkyl bedeutet,
- R14: einen durch R9 und R10 substituierten Cyclus bedeutet, der ausgewählt ist aus der Gruppe bestehend aus Benzol und Thiophen,
- R15: Carboxy bedeutet,
- W: CH oder N bedeutet,
- X: S bedeutet,
- Z: S bedeutet,
- m: eine Zahl von 1 bis 3 bedeutet,
- n: die Zahl 0 bedeutet,
- t: die Zahl 0 bedeutet,
- u: die Zahl 0, 1 oder 2 bedeutet,
- v: die Zahl 0 oder 1 bedeutet,
- p: die Zahl 3 bedeutet und
- q: die Zahl 0 oder 1 bedeutet
und ihre Salze,
wobei
- v: nicht die Zahl 1 bedeutet, wenn m die Zahl 1 bedeutet,
und wobei
- R4: nicht -N(R7)R8 oder einen über N (Stickstoff) gebundenen Cyclus oder Bicyclus bedeutet, wenn Z S bedeutet, v die Zahl 1 bedeutet und u die Zahl 0 bedeutet.

Eine Ausgestaltung der Erfindung (Ausgestaltung a) sind jene Verbindungen bzw. jene hervorzuhebenden, besonders hervorzuhebenden und beispielhaften Verbindungen der Formel I, worin t die Zahl 0 bedeutet und v die Zahl 0 bedeutet, und ihre Salze.

Eine weitere Ausgestaltung der Erfindung (Ausgestaltung b) sind jene Verbindungen bzw. jene hervorzuhebenden, besonders hervorzuhebenden und beispielhaften Verbindungen der Formel I, worin t die Zahl 0 bedeutet, v die Zahl 1 bedeutet und u die Zahl 0 bedeutet, und ihre Salze.

Eine weitere Ausgestaltung der Erfindung (Ausgestaltung c) sind jene Verbindungen bzw. jene hervorzuhebenden, besonders hervorzuhebenden und beispielhaften Verbindungen der Formel I, worin t die Zahl 0 bedeutet, v die Zahl 1 bedeutet und u die Zahl 1 oder 2 bedeutet, und ihre Salze.

Beispielhafte erfindungsgemäße Verbindungen sind in den folgenden Tabellen aufgeführt:

**Tabelle 1**

| Verbindungen der Formel I (siehe beigefügtes Formelblatt) mit W=CH, R=H, R1=H, R2=H, t=0, n=0, Anbindung des Pyridinringes in 2-Position, R4=Phenyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH3 | H | H | N-CH₃ | 2 | - | - | 0 |
| CH3 | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH3 | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 2**

| Verbindungen der Formel I (siehe beigefügtes Formelblatt) mit W=CH, R=H, R1=H, R2=H, t=0, n=0, Anbindung des Pyridinringes in 2-Position, R4=2-Furyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 3**

| Verbindungen der Formel I (siehe beigefügtes Formelblatt) mit W=CH, R=H, R1=H, R2=H, t=0, n=0, Anbindung des Pyridinringes in 2-Position, R4=4-Methyl-5-thiazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 4**

| Verbindungen der Formel I (siehe beigefügtes Formelblatt) mit W=CH, R=H, R1=H, R2=H, t=0, n=0, Anbindung des Pyridinringes in 2-Position, R4=1-Methyl-5-tetrazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 2 | S S | 1 1 | 1 1 |
| CH₃ | H | H | N-CH₃ | 3 | S | 1 | 1 |

**Tabelle 5**

| Verbindungen der Formel I (siehe beigefügtes Formelblatt) mit W=CH, R=H, R1=H, R2=H, t=0, n=0, Anbindung des Pyridinringes in 2-Position, R4=4-Pyridinyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH3 | 2 | S | 1 | 1 |
| CH₃ | H | H | N-CH₃ | 3 | S | 1 | 1 |

**Tabelle 6**

| Verbindungen der Formel I (siehe beigefügtes Formelblatt) mit W=CH, R=H, R1=H, R2=H, t=0, n=0, Anbindung des Pyridinringes in 2-Position, R4=4-Benzyl-1-piperazinyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | 1 |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 7**

| Verbindungen der Formel I (siehe beigefügtes Formelblatt) mit W=CH, R=H, R1=H, R2=H, t=0, n=0, Anbindung des Pyridinringes in 2-Position, R4=4-Phenyl-1-piperazinyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | 1 |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 8**

| Verbindungen der Formel I (siehe beigefügtes Formelblatt) mit W=CH, R=H, R1=H, R2=H, t=0, n=0, Anbindung des Pyridinringes in 2-Position, R4=1,2,3,4-Tetrahydroisochinolin-2-yl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | 1 |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 9**

| Verbindungen der Formel I (siehe beigefügtes Formelblatt) mit W=CH, R=H, R1=H, R2=H, t=0, n=0, Anbindung des Pyridinringes in 2-Position, R4=Dimethylthiocarbamoyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | N-CH₃ | 0 | 1 |
| CH₃ | H | H | S | 3 | N-CH₃ | 0 | 1 |
| CH₃ | CH₃ | H | S | 3 | N-CH₃ | 0 | 1 |
| OCH₃ | H | H | S | 3 | N-CH₃ | 0 0 | 1 1 |
| CH₃ | H | CH₃ | S | 3 | N-CH₃ | 0 | 1 |
| CH₃ | H | H | 0 | 2 | N-CH₃ | 0 | 1 |
| CH₃ | H | H | 0 | 3 | N-CH₃ | 0 | 1 |
| CH₃ | H | H | N-CH₃ | 2 | N-CH₃ | 0 | 1 |
| CH₃ | H | H | N-CH₃ | 3 | N-CH₃ | 0 | 1 |

**Tabelle 10**

| Verbindungen der Formel I (siehe beigefügtes Formelblatt) mit W=CH, R=H, R1=H, R2=H, t=0, n=0, Anbindung des Pyridinringes in 2-Position, R4=1-Imidazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 11**

| Verbindungen der Formel I (siehe beigefügtes Formelblatt) mit W=CH, R=H, R1=H, R2=H, t=0, n=0, Anbindung des Pyridinringes in 2-Position, R4=4-(5-Chlor-2-thienyl-methyl)-1-piperazinyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | 1 |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 12**

| Verbindungen der Formel I (siehe beigefügtes Formelblatt) mit W=CH, R=H, R1=H, R2=H, t=0, n=0, Anbindung des Pyridinringes in 2-Position, R4=2-Benzimidazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 13**

| Verbindungen der Formel I (siehe beigefügtes Formelblatt) mit W=CH, R=H, R1=H, R2=H, t=0, n=0, Anbindung des Pyridinringes in 2-Position, R4=2-Methoxycarbonylphenyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 14**

| Verbindungen der Formel I (siehe beigefügtes Formelblatt) mit W=CH, R=H, R1=H, R2=H, t=0, n=0, Anbindung des Pyridinringes in 2-Position, R4=2-Methyl-5-nitro-1-imidazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | 1 |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 15**

| Verbindungen der Formel I (siehe beigefügtes Formelblatt) mit W=CH, R=H, R1=H, R2=H, t=0, n=0, Anbindung des Pyridinringes in 2-Position, R4=5-Chlor-2-thienyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 16**

| Verbindungen der Formel I (siehe beigefügtes Formelblatt) mit W=CH, R=H, R1=H, R2=H, t=0, n=0, Anbindung des Pyridinringes in 2-Position, R4=2-Pyridin-3-carbonsäure und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | 5 | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 17**

| Verbindungen der Formel I (siehe beigefügtes Formelblatt) mit W=CH, R=H, R1=H, R2=H, t=0, n=0, Anbindung des Pyridinringes in 2-Position, R4=2-Thiazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 18**

| Verbindungen der Formel I (siehe beigefügtes Formelblatt) mit W=CH, R=H, R1=H, R2=H, t=0, n=0, Anbindung des Pyridinringes in 2-Position, R4=2-Imidazolyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

**Tabelle 19**

| Verbindungen der Formel I (siehe beigefügtes Formelblatt) mit W=CH, R=H, R1=H, R2=H, t=0, n=0, Anbindung des Pyridinringes in 2-Position, R4=5-Nitro-1-imidazolyl und den folgenden weiteren Substituenten- und Symbolbedeutungen: | | | | | | | |
|---|---|---|---|---|---|---|---|
| R3 | R5 | R6 | X | m | *l* | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | Q |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 2 | 1 |
| CH₃ | H | H | S | 3 | S | 2 | 1 |
| CH₃ | CH₃ | H | S | 3 | S | 2 | 1 |
| OCH₃ | H | H | S | 3 | S | 2 | 1 |
| CH₃ | H | CH₃ | S | 3 | S | 2 | 1 |
| CH₃ | H | H | 0 | 2 | S | 2 | 1 |
| CH₃ | H | H | 0 | 3 | S | 2 | 1 |
| CH₃ | H | H | N-CH₃ | 2 | S | 2 | 1 |
| CH₃ | H | H | N-CH₃ | 3 | S | 2 | 1 |
| CH₃ | H | H | S | 2 | S | 3 | 1 |
| CH₃ | H | H | S | 3 | S | 3 | 1 |
| CH₃ | CH₃ | H | S | 3 | S | 3 | 1 |
| OCH₃ | H | H | S | 3 | S | 3 | 1 |
| CH₃ | H | CH₃ | S | 3 | S | 3 | 1 |
| CH₃ | H | H | 0 | 2 | S | 3 | 1 |
| CH₃ | H | H | 0 | 3 | S | 3 | 1 |
| CH₃ | H | H | N-CH₃ | 2 | S | 3 | 1 |
| CH₃ | H | H | N-CH₃ | 3 | S | 3 | 1 |

**Tabelle 20**

| Verbindungen der Formel I (siehe beigefügtes Formelblatt) mit W=CH, R=H, R1=H, R2=H, t=0, n=0, Anbindung des Pyridinringes in 2-Position, R4=2-Pyridinyl und den folgenden weiteren Substituenten- und Symbolbedeutungen: | | | | | | | |
|---|---|---|---|---|---|---|---|
| R3 | R5 | R6 | X | m | Z | u | v |
| CH₃ | H | H | S | 1 | - | - | 0 |
| CH₃ | H | H | S | 2 | - | - | 0 |
| CH₃ | H | H | S | 3 | - | - | 0 |
| CH₃ | CH₃ | H | S | 3 | - | - | 0 |
| OCH₃ | H | H | S | 3 | - | - | 0 |
| CH₃ | H | CH₃ | S | 3 | - | - | 0 |
| CH₃ | H | H | 0 | 2 | - | - | 0 |
| CH₃ | H | H | 0 | 3 | - | - | 0 |
| CH₃ | H | H | N-CH₃ | 2 | - | - | 0 |
| CH₃ | H | H | N-CH₃ | 3 | - | - | 0 |
| CH₃ | H | H | S | 2 | S | 0 | 1 |
| CH₃ | H | H | S | 3 | S | 0 | 1 |
| CH₃ | CH₃ | H | S | 3 | S | 0 | 1 |
| OCH₃ | H | H | S | 3 | S | 0 | 1 |
| CH₃ | H | CH₃ | S | 3 | S | 0 | 1 |
| CH₃ | H | H | 0 | 2 | S | 0 | 1 |
| CH₃ | H | H | 0 | 3 | S | 0 | 1 |
| CH₃ | H | H | N-CH₃ | 2 | S | 0 | 1 |
| CH₃ | H | H | N-CH₃ | 3 | S | 0 | 1 |
| CH₃ | H | H | S | 2 | S | 1 | 1 |
| CH₃ | H | H | S | 3 | S | 1 | 1 |
| CH₃ | CH₃ | H | S | 3 | S | 1 | 1 |
| OCH₃ | H | H | S | 3 | S | 1 | 1 |
| CH₃ | H | CH₃ | S | 3 | S | 1 | 1 |
| CH₃ | H | H | 0 | 2 | S | 1 | 1 |
| CH₃ | H | H | 0 | 3 | S | 1 | 1 |
| CH₃ | H | H | N-CH₃ | 2 | S | 1 | 1 |
| CH₃ | H | H | N-CH₃ | 3 | S | 1 | 1 |

### Tabelle 21 - Tabelle 40

Verbindungen der Formel I (siehe beigefügtes Formelblatt) wie definiert in den Tabellen 1 - 20, aber mit Anbindung des Pyridin-Ringes in 4-Position,
und die Salze der Verbindungen in den Tabellen 1 - 40.

Weiterer Gegenstand der Erfindung ist ein Verfahren zur Herstellung der Verbindungen der Formel I und ihrer Salze.

Das Verfahren ist dadurch gekennzeichnet,
a) daß man Mercaptoverbindungen der Formel II (siehe beigefügtes Formelblatt), worin R und W die oben angegebenen Bedeutungen haben, mit Pyridinderivaten III (siehe beigefügtes Formelblatt), worin R1, R2, R3, R4, R5, R6, X, Y, Z, m, r, t, u und v die oben angegebenen Bedeutungen haben und A eine geeignete Abgangsgruppe darstellt, umsetzt, oder
b) daß man Verbindungen der Formel IV (siehe beigefügtes Formelblatt), worin W, R, R1, R2, R3, R5, R6, X, m und n die oben angegebenen Bedeutungen haben und A eine geeignete Abgangsgruppe darstellt, mit Verbindungen der Formel V (siehe beigefügtes Formelblatt), worin R4, Y, Z, r, t, u und v die oben angegebenen Bedeutungen haben, umsetzt, und
(falls Verbindungen der Formel I mit n=1 oder 2 und/oder Y=SO oder SO₂ und/oder Z=SO oder SO₂ die gewünschten Endprodukte sind), daß man anschließend die erhaltenen Verbindungen mit n=0 und/oder Y=S und/oder Z=S oxydiert, und/oder daß man erhaltene Verbindungen gewünschtenfalls anschließend in die Salze überführt und/oder daß man erhaltene Salze gewünschtenfalls anschließend in die freien Verbindungen überführt.

Bei den vorstehend aufgeführten Umsetzungen können die Ausgangsverbindungen als solche oder gegebenenfalls in Form ihrer Salze eingesetzt werden.

Als geeignete Abgangsgruppen A seien beispielsweise Halogenatome, insbesondere Chlor, oder durch Veresterung (z.B. mit p-Toluolsulfonsäure) aktivierte Hydroxylgruppen genannt.

Die Umsetzung von II mit III erfolgt in geeigneten, vorzugsweise polaren protischen oder aprotischen Lösungsmitteln (wie Methanol, Ethanol, Isopropanol, Dimethylsulfoxid, Aceton, Dimethylformamid oder Acetonitril) in Gegenwart oder unter Ausschluß von Wasser. Sie wird beispielsweise in Gegenwart eines Protonenakzeptors, gewünschtenfalls unter Zusatz katalytischer Mengen eines Jodids, wie Natriumjodid, durchgeführt. Als Protonenakzeptoren eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen 0° und 150°C liegen, wobei in Gegenwart von Protonenakzeptoren Temperaturen zwischen 20° und 80°C und ohne Protonenakzeptoren zwischen 60° und 120°C - insbesondere die Siedetemperatur der verwendeten Lösungsmittel - bevorzugt sind. Die Reaktionszeiten liegen zwischen 0,5 und 30 Stunden.

Die Umsetzung der Verbindungen IV mit den Verbindungen V erfolgt auf ähnliche Weise wie die Umsetzung der Verbindungen II mit den Verbindungen III bzw. alternativ [z.B. bei der Umsetzung der Verbindungen IV mit Verbindungen V, in denen t und v die Zahl 0 bedeutet und R4 den Rest N(R7)R8 darstellt] ohne zusätzliches Lösungsmittel unter Verwendung eines Überschusses an Amin als Protonenakzeptor und gleichzeitig Lösungsmittel. Die Reaktionstemperatur liegt in diesem Fall zwischen 60° und 180°C, bevorzugt zwischen 80° und 160°C.

Die Oxidation der Sulfide zu den Sulfoxiden bzw. Sulfonen erfolgt unter den Bedingungen, wie sie dem Fachmann für die Oxidation von Sulfiden zu Sulfoxiden bzw. Sulfonen geläufig sind [siehe hierzu z.B. J. Drabowicz und M. Mikolajczyk, Organic preparations and procedures int. 14(1-2), 45-89(1982) oder E. Block in S. Patai, The Chemistry of Functional Groups, Supplement E. Part 1, S. 539-608, John Wiley and Sons (Interscience Publication), 1980]. Als Oxidationsmittel kommen alle für die Oxidation von Sulfiden zu Sulfoxiden bzw. Sulfonen üblicherweise verwendeten Reagenzien in Frage.

Die erfindungsgemäßen Sulfoxide sind optisch aktive Verbindungen. Je nach Art der Substituenten können noch weitere Chiralitätszentren im Molekül sein. Die Erfindung umfaßt daher sowohl die Enantiomeren und Diastereomeren als auch ihre Mischungen und Racemate. Die Enantiomeren können in an sich bekannter Weise (beispielsweise durch Herstellung und Trennung entsprechender diastereoisomerer Verbindungen) separiert werden.

Die Ausgangsverbindungen II, III, IV und V sind bekannt oder sie können auf an sich bekannte Weise, z.B. analog dazu wie in den nachfolgenden Beispielen beschrieben, hergestellt werden.

Die folgenden Beispiele erläutern die Erfindung näher, ohne sie einzuschränken. Die erfindungsgemäßen Verbindungen und die Ausgangsverbindungen können auf analoge Weise wie in den Beispielen beschrieben hergestellt werden. Die Abkürzung RT steht für Raumtemperatur, h steht für Stunde(n), Schmp. für Schmelzpunkt, Zers. für Zersetzung.

### Beispiele

### Endprodukte

### 1. 4-(2-Furylmethylthio)-3-methyl-2-[(2-pyridinylthio)methyl]-pyridin

Zu einer Lösung von 2-Mercapto-pyridin (1,12 g/10 mMol) in 40 ml Ethanol und 21 ml 1 N Natronlauge wird ein Äquivalent (2,92 g) 2-Chlormethyl-4-(2-furylmethylthio)-3-methylpyridin-hydrochlorid (gelöst in 10 ml Wasser) innerhalb von 20 Min. bei 40°C zugetropft. Man rührt anschließend 2-3 h bei 50-60°C und weitere 3-4 h bei RT.

Man saugt vom ausgefallenen Feststoff ab, rührt mit Ethanol/Wasser (1:1) aus und trocknet im Vakuum. Man erhält die Titelverbindungen als ockerfarbenes Pulver; Schmp. 102-104°C; Ausb.: 91 % d.Th.

### 2. 4-Benzylthio-3-methyl-2-[(2-pyridinylthio)methyl]-pyridin

Nach der in Beispiel 1 beschriebenen Arbeitsweise erhält man durch Umsetzung von 2-Mercaptopyridin mit 4-Benzylthio-2-chlormethyl-3-methylpyridinhydrochlorid die Titelverbindung vom Schmp. 106-108°C.

### 3. 3-Methyl-4-[2-(4-methyl-5-thiazolyl)-ethylthio]-2-[(2-pyridinylthio)-methyl]-pyridin

Nach der in Beispiel 1 beschriebenen Arbeitsweise erhält man durch Umsetzung von 2-Mercaptopyridin mit 2-Chlormethyl-4-[2-(4-methyl-5-thiazolyl)-ethylthio]-3-methylpyridin-hydrochlorid die Titelverbindung als gelbes Öl. Man extrahiert mit Dichlormethan, wäscht mit Wasser, trocknet über Kalziumcarbonat und engt ein. Kristallisation aus Diisopropylether liefert die Titelverbindung; Schmp. 67-69°C.

### 4. 4-(2-Furylmethylthio)-3-methoxy-2-[(2-pyridinylthio)methyl]pyridin-dihydrochlorid

Nach der in Beispiel 1 angegebenen Arbeitsweise erhält man durch Umsetzung von 2-Mercaptopyridin mit 2-Chlormethyl-4-(2-furylmethylthio)-3-methoxypyridin-hydrochlorid ein gelbes Öl. Man extrahiert in Dichlormethan, engt ein, löst den Rückstand in Isopropanol und setzt konz. Salzsäure (2,5 Äquivalente) zu. Man engt wieder ein und kristallisiert durch Zugabe von Aceton die Titelverbindung als farblosen Feststoff; Schmp. 148°C (Zers.); Ausb. 74 % d.Th.

### 5. 3-Methyl-4-[5-(1-methyl-5-tetrazolyl)-1,5-dithiapentyl]-2-[(2-pyridinylthio-methyl]-pyridin

4-(3-Chlorpropylthio)-3-methyl-2-[(2-pyridinylthio)methyl]-pyridin-dihydrochlorid (0,59 g, 1,5 mMol) werden unter Zusatz von 1 N Natronlauge (5 mMol) mit 5-Mercapto-1-methyltetrazol (2,0 mMol) in Ethanol (15 ml) 24 h bei 80°C gerührt. Man tropft langsam Wasser zu, läßt auf 25°C abkühlen und filtriert vom ausgefallenen Feststoff. Nach Trocknung über P₂O₅ erhält man die Titelverbindung als hellbeigen Feststoff; Schmp. 113-114°C; Ausb. 78 % d.Th.

### 6. 3-Methyl-4-[5-(4-pyridinyl)]-1,5-dithiapentyl]-2-[(2-pyridinylthio)methyl]-pyridin

Nach der in Beispiel 5 angegebenen Arbeitsweise erhält man durch Umsetzung von 4-(3-Chlorpropylthio)-3-methyl-2-[(2-pyridinylthio)methyl]-pyridin-dihydrochlorid mit 4-Mercaptopyridin und Natronlauge die Titelverbindung; Schmp. 99-102°C; Ausb. 67 % d.Th.

### 7. 4-[3-(4-Benzyl-1-piperazinyl)-propylthio]-3-methyl-2-[(2-pyridinylthio)methyl]-pyridin-hydrochloridsalz

4-(3-Chlorpropylthio)-3-methyl-2-[(2-pyridinylthio)methyl]-pyridin-dihydrochlorid (0,59 g; 1,5 mMol) wird in Acetonitril (10 ml) unter Zusatz von Kaliumcarbonat (7,5 mMol) und katalytischen Mengen von Natriumjodid mit Benzylpiperazin (2,0 mMol) 24 h bei 100°C gerührt. Nach Zugabe von Wasser wird mit Dichlormethan (2 x 10 ml) extrahiert, die vereinigten organischen Phasen mit Wasser gewaschen, getrocknet, eingeengt und das Rohprodukt (gelbes Öl) an Kieselgel chromatographiert. Die reinen Produktfraktionen werden vereinigt, eingeengt, in Ethanol gelöst und mit 3,5 Äquivalenten konz. Salzsäure versetzt. Man filtriert vom ausgefallenen Feststoff, wäscht mit Diisopropylether nach und trocknet. Man erhält die Titelverbindung als farblose Kristalle; Schmp. 170-172°C; Ausb. 81 % d.Th.

### 8. 3-Methyl-4-[3-(4-phenyl-1-piperazinyl)-propylthio]-2-[(2-pyridinylthio)methyl]-pyridin-hvdrochloridsalz

Nach der in Beispiel 7 beschriebenen Arbeitsweise erhält man durch Umsetzung von 4-(3-Chlorpropylthio)-3-methyl-2-[(2-pyridinylthio)methyl]-pyridin-dihydrochlorid mit 1-Phenylpiperazin und Kaliumcarbonat die Titelverbindung; Schmp. 137-140°C (Zers.); Ausb. 46 % d.Th.

### 9. 3-Methyl-4-[3-(1,2,3,4-tetrahydro-isochinolin-2-yl)-propylthio]-2-[(2-pyridinylthio)methyl]-pyridin

Nach der in Beispiel 7 beschriebenen Arbeitsweise erhält man durch Umsetzung von 4-(3-Chlorpropylthio)-3-methyl-2-[(2-pyridinylthio)methyl]-pyridin-dihydrochlorid mit 1,2,3,4-Tetrahydroisochinolin und Kaliumcarbonat die Titelverbindung; Schmp. hygroskopisch; ab 58°C Zers.; Ausb. 46 % d.Th.

### 10. 3-Methyl-4-{3-[4-(3-phenyl-2-propen-1-yl)-piperazin-1-yl]-propylthio} 2-[(2-pyridinylthio)methyl]-pyridin-hydrochloridsalz

Nach der in Beispiel 7 beschriebenen Arbeitsweise erhält man durch Umsetzung von 4-(3-Chlorpropylthio)-3-methyl-2-[(2-pyridinylthio)methyl]-pyridin-dihydrochlorid mit N-3-Phenyl-2-propenylpiperazin die Titelverbindung; Schmp. 205-206°C (Zers.); Ausb. 69 % d.Th.

### 11. 4-(2-Furylmethylthio)-3-methyl-2-[(4-pyridinylthio)methyl]-pyridin

Nach der im Beispiel 1 angegebenen Arbeitsweise erhält man durch Umsetzung von 4-Mercaptopyridin mit 2-Chlormethyl-4-(2-furylmethylthio)-3-methylpyridin-hydrochlorid und Natronlauge, nachfolgender Chromatographie an Kieselgel (Ethylacetat/Ethanol) und Kristallisation aus Diisopropylether die Titelverbindung als farbloses Pulver; Schmp. 126-128°C; Ausb. 89 % d.Th.

### 12. 3-Methyl-4-[5-(1-methyl-5-tetrazolyl)]-1,5-dithiapentyl]-2-[(4-pyridinylthio)methyl]-pyridin

Nach der im Beispiel 5 angegebenen Arbeitsweise erhält man durch Umsetzung von 4-(3-Chlorpropylthio)-3-methyl-2-[(4-pyridinylthio)methyl)-pyridin mit 5-Mercapto-1-methyltetrazol und Natronlauge die Titelverbindung als beiges Pulver; Schmp. 95-97°C, Zers., Ausb. 59 % d.Th.

### 13. 4-[3-(4-Benzyl-1-piperazinyl)-propylthio]-3-methyl-2-[(4-pyridinylthio)methyl]-pyridin

Nach der in Beispiel 7 angegebenen Arbeitsweise erhält man durch Umsetzung von 4-(3-Chlorpropylthio)-3-methyl-2-[(4-pyridinylthio)methyl]-pyridin mit 1-Benzylpiperazin und Kaliumcarbonat in Acetonitril nach Chromatographie an Kieselgel und Kristallisation der eingeengten reinen Fraktion aus Diisopropylether die Titelverbindung als farblosen Feststoff; Schmp. 79-81°C; Ausb. 57 % d.Th. Aus Isopropanol läßt sich ein wasserhaltiges Hydrochlorid herstellen; Schmp. 165°C (Zers.); Ausb. 87 % d.Th.

### 14. 3-Methyl-2-[(4-pyridinylthio)methyl]-4-[5-(4-pyridinyl)-1,5-dithiapentyl]-pyridin

Nach der in Beispiel 5 angegebenen Arbeitsweise erhält man durch Umsetzung von 4-(3-Chlorpropylthio)-3-methyl-2-[(4-pyridinylthio)methyl]-pyridin mit 4-Mercaptopyridin und Natronlauge die Titelverbindung; Schmp. 116-118°C; Ausb. 69 % d.Th.

### 15. 4-[(3-Dimethyldithiocarbamoyl)propylthio]-3-methyl-2-[(4-pyridinylthio)methyl]-pyridin

4-(3-Chlorpropylthio)-3-methyl-2-[(4-pyridinylthio)methyl]pyridin (2 mMol) werden mit Dimethyldithiocarbamidsäure-Na-Salz (2,5 mMol) in 25 ml Ethanol 20 h bei 60°C gerührt, abgekühlt und vom entstandenen Feststoff filtriert. Man erhält die Titelverbindung als hellgraues, kristallines Pulver; Schmp. 112-114°C; Verfärbung; Ausb. 88 % d.Th.

### 16. 4-[3-(4-Phenyl-1-piperazinyl)-propylthio]-2-[(4-pyridinylthio)methyl]-pyridin

Nach der in Beispiel 7 angegebenen Arbeitsweise erhält man durch Umsetzung von 4-(3-Chlorpropylthio)-3-methyl-2-[(4-pyridinylthio)methyl]-pyridin mit 1-Phenylpiperazin und Kaliumcarbonat und nachfolgende Chromatographie an Kieselgel nach Kristallisation aus Diisopropylether die Titelverbindung; Schmp. ab 210°C (Zers.); Ausb. 79 % d.Th.

### 17. 4-[3-(1-Imidazolyl)-propylthio]-3-methyl-2-[(4-pyridinylthio)methyl]-pyridin

Nach der in Beispiel 7 angegebenen Arbeitsweise erhält man durch Umsetzung von 4-(3-Chlorpropylthio)-3-methyl-2-[(4-pyridinylthio)methyl]-pyridin mit Imidazol (2,0 Äquivalente) und Kaliumcarbonat und nachfolgende Chromatographie an Kieselgel (Dichlormethan/Aceton/NH₃aq) nach Kristallisation aus Diisopropylether die Titelverbindung; Schmp. 117-119°C; Ausb. 32 % d.Th.

### 18. 3-Methyl-4-[3-(1,2,3,4-tetrahydroisochinolin-1-yl)-propylthio]-2-[(4-pyridinylthio)methyl]-pyridin

Nach der in Beispiel 7 angegebenen Arbeitsweise erhält man durch Umsetzung von 4-(3-Chlorpropylthio)-3-methyl-2-[(4-pyridinylthio)methyl]-pyridin mit 1,2,3,4-Tetrahydroisochinolin, Chromatographie an Kieselgel und anschliessende Kristallisation aus Isopropanol/Diisopropylether die Titelverbindung; Schmp. 190-192°C; Ausb. 36 % d.Th.

### 19. 4-{3-[4-(5-Chlor-2-thienylmethyl)-1-piperazinyl]-propylthio)-3-methyl-2-[(4-pyridinylthio)methyl]-pyridin-trihydrochlorid

Nach der in Beispiel 7 angegebenen Arbeitsweise erhält man durch Umsetzung von 4-(3-Chlorpropylthio)-3-methyl-2-[(4-pyridinylthio)methyl]-pyridin mit [1-(5-Chlorthiophen-2-yl)methyl]-piperazin und Kaliumcarbonat nach Kristallisation aus Isopropanol/Aceton/konz. Salzsäure die Titelverbindung; Schmp. 160-162°C Zers.; Ausb. 79 % d.Th.

### 20. 2-[[[3-Methyl-2-[(4-pyridinylthio)methyl]-4-pyridinyl]thiopropyl]thio]-1H-benzimidazol

Nach der in Beispiel 5 angegebenen Arbeitsweise erhält man durch Umsetzung von 4-(3-Chlorpropylthio)-3-methyl-2-[(4-pyridinylthio)methyl]-pyridin mit 2-Mercaptobenzimidazol in Gegenwart von Natronlauge nach Kristallisation aus Dichlormethan/Diisopropylether die Titelverbindung; Schmp. 128-129°C; Ausb. 83 % d.Th.

### 21. 4-[[5-(2-Methoxycarbonyl-phenyl)-1,5-dithiapent-1-yl]-3-methyl]-2-[(4-pyridinylthio)methyl]-pyridin

4-(3-Chlorpropylthio)-3-methyl-2-[(4-pyridinylthio)methyl]pyridin (2 mMol) wird unter Zusatz von Kaliumcarbonat (10 mMol) mit 2-Mercaptobenzoesäuremethylester (2,2 mMol) 48 h bei 25°C in Methanol (10 ml) gerührt, mit Wasser verdünnt, vom ausgefallenen Feststoff filtriert und aus Methanol/Wasser ausgerührt. Nach Trocknung erhält man die Titelverbindung als beiges Pulver; Schmp. 85-88°C; Ausb. 72 % d.Th.

### 22. 3-Methyl-4-[3-(2-methyl-5-nitro-imidazo]-1-yl)-propylthio]-2-[(4-pyridinylthio)methyl]-pyridin

Nach der im Beispiel 7 angegebenen Arbeitsweise erhält man ausgehend von 4-(3-Chlorpropylthio)-3-methyl-2-[(4-pyridinylthio)methyl]pyridin mit 2-Methyl-5-nitroimidazol, nachfolgende Kieselgelchromatographie (Ethylacetat/Methanol/konz. Ammoniak) und Kristallisation aus Diisopropylether die Titelverbindung als gelbes Pulver; Schmp. 140-142°C; Ausb. 70 % d.Th.

### 23. 3-Methyl-4-(7-phenyl-1,5-dithiahept-1-yl)-2-[(4-pyridinylthio)methyl]-pyridin

Nach der im Beispiel 5 angegebenen Arbeitsweise erhält man durch Umsetzung von 4-(3-Chlorpropylthio)-3-methyl-2-((4-pyridinylthio)methyl]pyridin mit 2-Phenylethylmercaptan nach Kieselgelchromatographie und Kristallisation aus Diisopropylether die Titelverbindung als farbloses Pulver; Schmp. 48-50°C; Ausb. 49 % d.Th.

### 24. 4-[6-(5-Chlorthiophen-2-yl)-1,5-dithiahex-1-yl]-3-methyl-2-[(4-pyridinylthio)methyl]-pyridin

Nach der im Beispiel 5 angegebenen Arbeitsweise erhält man durch Umsetzung von 4-(3-Chlorpropylthio)-3-methyl-2-[(4-pyridinylthio)methyl]pyridin mit 5-Chlorthiophen-2-methylmercaptan nach Kieselgelchromatographie (Ethylacetat/konz. Ammoniak. 100/1) und anschließende Kristallisation aus Diisopropylether die Titelverbindung als farbloses Pulver; Schmp. 76-77°C; Ausb. 58% d.Th.

### 25. 2-{5-[3-Methyl-2-[(4-pyridinylthio)methyl]-4-pyridinyl]-1.5-dithiapentyl}-pyridin-3-carbonsäure

Nach der im Beispiel 21 angegebenen Arbeitsweise erhält man durch Umsetzung von 4-(3-Chlorpropylthio)-3-methyl-2-[(4-pyridinylthio)methyl]pyridin mit 2-Mercaptonicotinsäure, und anschließende Einstellung eines pH-Wertes von ca. 6 die Titelverbindung als farblosen Feststoff; Schmp. 219°C Zers.; Ausb. 57 % d.Th.

### 26. 6-Methyl-4-[5-(4-pyridinyl)]-1,5-dithiapentyl]-2-[(4-pyridinylthio)methyl]-pyridin-sesqui-fumarat

Nach der in Beispiel 5 angegebenen Arbeitsweise erhält man durch Umsetzung von 4-(3-Chlorpropylthio)-6-methyl-2-[(4-thio-pyridinyl)methyl]-pyridin-dihydrochlorid mit 4-Mercaptopyridin und Natronlauge nach Chromatographie des Rohproduktes an Kieselgel (Fließmittel: Essigester/Methanol/Ammoniak = 40:1:1) und anschließender Kristallisation mit 1,5 Äquivalenten Fumarsäure aus Aceton die Titelverbindung (Ausb. 27 % d.Th.) vom Schmp. 150-152°C.

### 27. 4-[3-(4-Benzyl-1-piperazinyl)-propylthio]-6-methyl-2-[(4-pyridinylthio)methyl]-pyridin-difumarat

Nach der in Beispiel 7 angegebenen Arbeitsweise erhält man durch Umsetzung von 4-(3-Chlorpropylthio)-6-methyl-2-[(4-thio-pyridinyl)methyl]-pyridin-dihydrochlorid mit 1-Benzylpiperazin, Natriumjodid und Kaliumcarbonat in Acetonitril nach Chromatographie des Rohproduktes an Kieselgel (Fließmittel: Essigester/Methanol/Ammoniak = 19:1:1) und anschließender Kristallisation mit 2 Äquivalenten Fumarsäure aus Aceton die Titelverbindung (Ausb. 14 % d.Th.) vom Schmp. 171-173°C.

### 28. 4-{3-[4-(5-Chlorthienylmethyl)-1-piperazinyl]-propylthio)-6-methyl-2-[(4-pyridinylthio)methyl]-pyridin-difumarat

Nach der in Beispiel 7 angegebenen Arbeitsweise erhält man durch Umsetzung von 4-(3-Chlorpropylthio)-6-methyl-2-[(4-thio-pyridinyl)methyl]-pyridin-dihydrochlorid mit 1-[(5-Chlorthiophen-2-yl)methyl]-piperazin, Natriumjodid und Kaliumcarbonat in Acetonitril nach Chromatographie des Rohproduktes an Kieselgel (Fließmittel: Essigester/Methanol/Ammoniak = 19:1:1) und anschließender Kristallisation mit 2 Äquivalenten Fumarsäure aus Aceton die Titelverbindung (Ausb. 38 % d.Th.) vom Schmp. 148-151°C.

### 29. 4-[[7-(2-Methyl-5-nitro-imidazol-1-yl)-1,5-dithiahept-1-yl]-3-methyl]-2-[(4-pyridinyl-thio)methyl]-pyridin-dihydrochlorid

Nach der in Beispiel 5 beschriebenen Arbeitsweise erhält man durch Umsetzung mit 1-(2-Mercaptoethyl)-2-methyl-5-nitro-imidazol bei 25°C, Chromatographie des Rohproduktes an Kieselgel und Überführung ins Hydrochloridsalz in Aceton/Salzsäure die hygroskopische Titelverbindung; Schmp.: 73-78°C; Zers; Ausb. 39 % d.Th.

### 30. 5-{5-[3-Methyl-2-[(4-pyridinylthio)methyl]-4-pyridinyl]-1,5-dithiapent-1-yl}tetrazol-1-essigsäure

Nach der in Beispiel 25 und 21 beschriebenen Arbeitsweise erhält man die Titelverbindung; Schmp.: 185-187°C; Ausb. 57 % d.Th.

### 31. 4-[3-(4-Benzyl-1-piperazinyl)-propylthio]-3-methyl-2-[(2-pyrimidinylthio)methyl]-pyridin-trihvdrochlorid

Ausgehend von 4-(3-Chlorpropylthio)-3-methyl-2-[(2-pyrimidinylthio)methyl]-pyridin-dihydrochlorid erhält man nach der in Beispiel 7 beschriebenen Arbeitsweise durch Umsetzung mit Benzylpiperazin die Titelverbindung; Schmp. 208°C; Zersetzung; Ausb. 49 % d.Th.

### 32. 3-Methyl-4-[3-(2-methyl-5-nitro-imidazol-1-yl)-propylthio]-2-[(2-pyrimidinylthio)methyl]-pyridin

Nach der in Beispiel 22 beschriebenen Arbeitsweise erhält man ausgehend von 4-(3-Chlorpropylthio)-3-methyl-2-[(2-pyrimidinylthio)methyl]pyridin die Titelverbindung; Schmp.: 141-143°C; Ausb. 81 % d.Th.

### 33. 3-Methyl-4-{[7-(2-methyl-5-nitro-imidazol-1-yl)-1,5-dithiahept-1-yl]-3-methyl}-2-[(2-pyrimidinylthio)methyl]-pyridin

Nach der in Beispiel 5 beschriebenen Arbeitsweise erhält man ausgehend von 4-(3-Chlorpropylthio)-3-methyl-2-[(2-pyrimidinylthio)methyl]-pyridin die Titelverbindung als gelbes Öl, das beim Verreiben mit Diethylether kristallisiert. Nach Filtration und Trocknung über Paraffin erhält man die Titelverbindung als blaßgelben Feststoff; Schmp. 83-85°C; Ausb.: 62 % d.Th.

### Ausgangsverbindungen

### A1. 4-(3-Chlorpropylthio)-3-methyl-2-[(2-pyridinylthio)methyl]-pyridin-dihydrochlorid

2-Mercaptopyridin (10 mMol) und 2-Chlormethyl-4-(3-chlorpropylthio)-3-methylpyridin-hydrochlorid (10 mMol) werden in Isopropanol (25 ml) 4-6 h zum Sieden erhitzt. Nach Abkühlung filtriert man vom ausgefallenen Feststoff, wäscht mit Isopropanol und trocknet im Vakuum bei 40°C. Man erhält 3,6 g (91 % d.Th.) der Titelverbindung als farblosen Feststoff; Schmp. 112-114°C, (Zers.).

### A2. 2-Chlormethyl-4-(3-chlorpropylthio)-3-methylpyridin-hydrochlorid

### a) 2,3-Dimethyl-4-(3-hydroxypropylthio)pyridin-N-oxid

Zu 50 ml trockenem N-Methylpyrrolidon (NMP) werden 6 g (60 %iges) NaH portionsweise zugegeben, es wird 15 Min. gerührt, 9,5 g (0,11 Mol) 3-Hydroxypropylmercaptan werden innerhalb von 20 Min. zudosiert und es wird erneut 30 Min. bis zur Beendigung der Gasentwicklung gerührt. Anschließend tropft man innerhalb von 20 Min. eine Lösung von 14,4 g (0,1 Mol) 4-Chlor-2,3-dimethylpyridin-N-oxid in 100 ml NMP zu, rührt die Reaktionsmischung 1 h bei RT, anschließend 1 h bei 70°C und danach noch 1 h bei 100°C.

Nach beendeter Umsetzung läßt man abkühlen, verdünnt mit 500 ml Wasser und extrahiert 4 mal mit je 300 ml Dichlormethan. Die vereinigten organischen Phasen werden mit Wasser gewaschen, über Magnesiumsulfat getrocknet, eingeengt und aus Toluol kristallisiert. Nach Umkristallisation aus Methanol/Toluol erhält man die Titelverbindung als beigen Feststoff vom Schmp. 106-107°C (sublimiert): Ausb. 68 % d.Th.

### b) 2-Hydroxymethyl-4-(3-hydroxypropylthio)-3-methylpyridin

Man löst das unter a) erhaltene gelbe Öl in 100 ml Essigsäureanhydrid, und rührt 2 h bei 100°C. Nach Einengen im Vakuum wird der braune, ölige Rückstand in einer Kugelrohrdestillationsapparatur destilliert und ohne Reinigung weiter umgesetzt.

Das ölige Destillat wird in 100 ml 2 N Natronlauge und 100 ml Isopropanol 2 h unter Rühren auf Rückflußtemperatur erhitzt, Isopropanol abdestilliert, der Rückstand 3 mal mit je 100 ml Dichlormethan extrahiert, die vereinigten organischen Phasen mit Wasser gewaschen, über Kaliumcarbonat getrocknet und im Vakuum eingeengt. Man erhält 5,0 g 2-Hydroxymethyl-4-(3-hydroxypropylthio)-3-methylpyridin, das ohne Reinigung weiter umgesetzt wird.

Aus Isopropanol läßt sich mit konz. Salzsäure ein Monohydrochlorid der Titelverbindung herstellen; Schmp. 188-190°C (Zers.).

### c) 2-Chlormethyl-4-(3-chlorpropylthio)-3-methylpyridin-hydrochlorid

5,0 g des Öls aus b) werden in Dichlormethan (100 ml) gelöst, 4 Äquivalente Thionylchlorid zugetropft und 20 h bei RT gerührt. Man engt vollständig ein und erhält 4,5 g der Titelverbindung als öligen, allmählich kristallisierenden Rückstand. Kristallisation aus Isopropanol/Diisopropylether liefert die Titelverbindung als farblosen Feststoff; Schmp. 142-144°C (Zers.).

### A3. 4-(3-Chlorpropylthio)-3-methyl-2-[(4-pyridinylthio)methyl]-pyridin

Nach der in Beispiel A1. beschriebenen Arbeitsweise erhält man durch Umsetzung von 4-Mercaptopyridin mit 2-Chlormethyl-4-(3-chlorpropylthio)-3-methyl-pyridin-hydrochlorid in Isopropanol ein Hydrochlorid der Titelverbindung als farblosen Feststoff. Nach Auflösung in Wasser wird ein pH von 10 eingestellt, 2 x mit Dichlormethan extrahiert, die organischen Phasen mit Natriumcarbonatlösung gewaschen, getrocknet (Magnesiumsulfat), das Lösungsmittel am Rotavapor eingeengt und aus Dichlormethan/Diisopropylether kristallisiert. Man erhält die Titelverbindung als farblosen Feststoff; Ausb. 78 % d.Th.; Schmp. 88-91°C.

B1. 4-(2-Chlorethylthio)-2-chlormethyl-3-methylpyridin-hydrochlorid

### a) 2.3-Dimethyl-4-(2-hydroxyethylthio)pyridin-N-oxid

Nach der in Beispiel A2. a) angegebenen Arbeitsweise erhält man durch Umsetzung von 4-Chlor-2,3-dimethylpyridin-N-oxid mit 2-Mercaptoethanol und Natriumhydrid die Titelverbindung als öligen Rückstand, der ohne weitere Reinigung in der Folgestufe eingesetzt wird.

### b) 4-(2-Hydroxyethylthio)-2-hydroxymethyl-3-methylpyridin

Nach der in Beispiel A2. b) angegebenen Arbeitsweise erhält man durch Umsetzung des unter a) erhaltenen Öls mit Essigsäureanhydrid und anschließender Verseifung mit NaOH die Titelverbindung als öligen Rückstand, der ohne weitere Reinigung in der Folgestufe eingesetzt wird.

### c) 4-(2-Chlorethylthio)-2-chlormethyl-3-methylpyridin-hydrochlorid

Nach der in Beispiel A2. c) angegebenen Arbeitsweise erhält man durch Umsetzung des unter b) erhaltenen Öls mit Thionylchlorid Titelverbindung als öligen Rückstand, der als Lösung in Ethanol direkt zur Umsetzung mit 2-Mercaptobenzimidazol eingesetzt wird.

### C1. 2-Chlormethyl-4-(3-chlorpropylthio)-3-methoxy-pyridin-hydrochlorid

Nach der im Beispiel A2. a)-c) beschriebenen Arbeitsweise erhält man ausgehend von 4-Chlor-3-methoxy-2-methylpyridin durch Umsetzung zuerst mit 3-Hydroxypropylmercaptan, anschließend aufeinanderfolgend mit Essigsäureanhydrid, Natronlauge und Thionylchlorid die Titelverbindung als gelbes, langsam kristallisierendes Öl, das direkt zur Umsetzung mit Mercaptopyridinen verwendet wird.

### D1. 3-Chlor-4-[N-(2-Chlorethyl)-N-methylamino]-2-[(2-pyridinylthio)methyl]-pvridin-dihydrochlorid

Nach der in Beispiel A1. beschriebenen Arbeitsweise erhält man durch Umsetzung von 3-Chlor-4-[N-(2-Chlorethyl)-N-methylamino]-2-chlormethyl-hydrochlorid mit 4-Mercaptopyridin (1 Äquivalent) in Isopropanol die Titelverbindung als farblosen Feststoff; Schmp. 208-210°C Zers. (Ausb. 81 % d.Th.).

### D2. 3-Chlor-4-[N-(2-chlorethyl)-N-methylamino]-2-chlormethylpyridin-hydrochlorid

### a) 3-Chlor-4-[N-(2-hydroxyethyl)-N-methylamino]-2-hydroxymethylpyridin

Eine Mischung aus 3,4-Dichlor-2-hydroxymethylpyridin (J.Med.Chem. 1989, 32, 1970) (2,5 g) in 2-Methylaminoethanol (30 ml) wird in einem Stahlautoklaven 2,5 h bei 160°C erwärmt, das überschüssige Amin im Hochvakuum abgezogen und der verbleibende Rückstand an Kieselgel chromatographiert (Dichlormethan/-Methanol 95/5). Ausbeute: 2,3 g als gelbliches Öl.

### b) 3-Chlor-4-[N-(2-chlorethyl)-N-methylamino]-2-chlormethylpyridin-hydrochlorid

Eine Lösung von 3-Chlor-4-[N-(2-hydroxyethyl)-N-methylamino]-2-hydroxy-methylpyridin (2,3 g) in Dichlormethan (30 ml) wird bei 0°C tropfenweise mit einer Lösung von Thionylchlorid (4 ml) in Dichlormethan (20 ml) versetzt. Anschließend läßt man die Temperatur auf 20°C steigen (20 Min.) und hält danach die Temperatur 30 Min. bei 40°C. Nach Abziehen des Lösungsmittels im Vakuum wird der verbleibende Rückstand an Kieselgel chromatographiert (Petrolether/Ethylacetat 7/3 Mischung, die 1 ml konz. NH₃ x aq/L enthält). Ausbeute: 2,6 g.

### E1. 4-(3-Chlorpropylthio)-6-methyl-2-[(4-thio-pyridinyl)methyl]-pyridindihydrochlorid

4-Mercaptopyridin (10,9 mMol) und 2-Chlormethyl-4-(3-chlorpropylthio)-6-methylpyridin-hydrochlorid (10,9 mMol) werden in Isopropanol (25 ml) 6 h zum Sieden erhitzt. Nach Abkühlen auf RT wird Methanol (25 ml) und Kieselgel (10 g) zugegeben, zur Trockne eingeengt und anschließend an Kieselgel (Fließmittel: Essigester/Methanol/Ammoniak = 19:1:1) chromatographiert. Die Fraktionen mit Rf = 0,3 werden eingeengt, in wenig Aceton gelöst und mit 2 Äquivalenten konz. Salzsäure versetzt. Der Niederschlag wird abgesaugt und im Hochvakuum getrocknet. Man erhält 3,55 g (82 % d.Th.) der Titelverbindung als beigen Feststoff; Schmp. 194-197°C.

### E2. 2-Chlormethyl-4-(3-chlorpropylthio)-6-methylpyridin-hydrochlorid

### a) 2,6-Dimethyl-4-(3-hydroxypropylthio)pyridin-N-oxid

Zu 50 ml trockenem N-Methylpyrrolidon (NMP) werden 12 g (60 %iges) NaH portionsweise zugegeben. Es wird 10 Min. gerührt. 19 g (0,22 Mol) 3-Hydroxy-propylmercaptan werden innerhalb von 30 Min. zudosiert und es wird bis zur Beendigung der Gasentwicklung weitere 30 Min. gerührt. Anschließend tropft man innerhalb von 30 Min. eine Lösung von 28,8 g (0,2 Mol) 4-Chlor-2,6-dimethylpyridin-N-oxid in 150 ml NMP zu, rührt die Reaktionsmischung 1 h bei RT, anschließend 1 h bei 70°C und danach noch 1 h bei 100°C.

Nach beendeter Umsetzung läßt man abkühlen, verdünnt mit 700 ml Wasser und extrahiert zunächst 4 mal mit je 300 ml Dichlormethan und anschließend noch 4 mal mit je 300 ml Dichlormethan/n-Butanol (10:1). Die vereinigten organischen Extrakte werden über Magnesiumsulfat getrocknet, eingeengt und aus Toluol kristallisiert. Die Titelverbindung wird als beiger Feststoff vom Schmp. 117-119°C isoliert. Ausb. 59 % d.Th.

### b) 2-Hydroxymethyl-4-(3-hydroxypropylthio)-6-methylpyridin

Man löst das unter a) erhaltene Produkt in 100 ml Acetanhydrid und rührt 2 h bei 100°C. Nach Einengen im Vakuum wird der braune, ölige Rückstand in einer Kugelrohrdestillationsapparatur destilliert und ohne Reinigung weiter umgesetzt.

Das ölige Destillat wird mit 100 ml 2 n Natronlauge und 100 ml Isopropanol 2 h am Rückfluß erhitzt. Isopropanol wird abdestilliert und der Rückstand 4 mal mit je 100 ml Dichlormethan und 4 mal mit je 100 ml Dichlormethan/n-Butanol (10:1) extrahiert. Die vereinigten organischen Extrakte werden mit Wasser gewaschen, über Kaliumcarbonat getrocknet und und im Vakuum eingeengt. Man erhält 4,2 g der Titelverbindung als Öl, das ohne Reinigung weiter umgesetzt wird.

Nach Chromatographie an Kieselgel (Fließmittel: Essigester/Methanol = 10:1) und anschließender Kristallisation aus Diisopropylether wird die Titelverbindung in kristalliner Form isoliert. Schmp. 94-96°C.

### c) 2-Chlormethyl-4-(3-chlorpropylthio)-6-methylpyridin-hydrochlorid

4,0 g des Öls aus b) werden in Dichlormethan (80 ml) gelöst, 4 Äquivalente Thionylchlorid zugetropft und 48 h bei RT gerührt. Man gibt 20 ml Toluol zu, engt vollständig ein, trocknet am Hochvakuum und erhält 5,3 g der Titelverbindung als gelbes Öl. 1H-NMR(DMSO-D6, delta ppm): 7.88 (d,1H), 7.77 (d,1H) 5.00 (s,2H), 3.79 (t,2H), 3,40 (t,2H), 2.70 (s,3H), 2,14 (m,2H).

### F1. 4-(3-Chlorpropylthio)-3-methyl-2-[(2-pyrimidinylthio)methyl]-pyridin

Nach der in Beispiel A3. beschriebenen Arbeitsweise erhält man durch Umsetzung mit 2-Mercaptopyrimidin die Titelverbindung; Schmp. 83-85°C; farbloses Pulver; Ausb. 73 % d.Th.

### Gewerbliche Anwendbarkeit

Die ausgezeichnete Wirksamkeit von Verbindungen der Formel I und ihren Salzen gegen Helicobacter-Bakterien gestattet ihren Einsatz in der Humanmedizin als Wirkstoffe für die Behandlung von Krankheiten, die auf Helicobacter-Bakterien beruhen.

Gegenstand der Erfindung sind außerdem die Verbindungen der Formel I und ihre pharmakologisch verträglichen Salze zur Anwendung bei der Behandlung von Krankheiten, die auf Helicobacter-Bakterien beruhen.

Ebenso umfaßt die Erfindung die Verwendung von Verbindungen der Formel I und ihren pharmakologisch verträglichen Salzen bei der Herstellung von Arzneimitteln, die zur Bekämpfung solcher Krankheiten eingesetzt werden, die auf Helicobacter-Bakterien beruhen.

Ein weiterer Gegenstand der Erfindung sind Arzneimittel zur Bekämpfung von Helicobacter-Bakterien, die eine oder mehrere Verbindungen der allgemeinen Formel I und/oder ihre pharmakologisch verträglichen Salze enthalten.

Von den Helicobacter-Stämmen, gegenüber denen sich die Verbindungen der Formel I als wirksam erweisen, sei insbesondere der Stamm Helicobacter pylori erwähnt.

Die Arzneimittel werden nach an sich bekannten, dem Fachmann geläufigen Verfahren hergestellt. Als Arzneimittel werden die pharmakologisch wirksamen Verbindungen der Formel I und ihre Salze (=Wirkstoffe) entweder als solche, oder vorzugsweise in Kombination mit geeigneten pharmazeutischen Hilfsstoffen z.B. in Form von Tabletten, Dragees, Kapseln, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder Permeationspromotoren und Komplexbildner (z.B. Cyclodextrine) verwendet werden.

Die Wirkstoffe können beispielsweise parenteral (z.B. intravenös) oder insbesondere oral appliziert werden.

Im allgemeinen werden in der Humanmedizin die Wirkstoffe in einer Tagesdosis von etwa 0,2 bis 50, vorzugsweise 1 bis 30 mg/kg Körpergewicht, gegebenenfalls in Form mehrerer, vorzugsweise 2 bis 6 Einzelgaben zur Erzielung des gewünschten Ergebnisses verabreicht.

In diesem Zusammenhang ist als erfindungswesentlicher Aspekt besonders zu erwähnen, daß sich die Verbindungen der Formel I, in denen n die Zahl 0 bedeutet, gegenüber Helicobacter-Bakterien bereits bei Verabfolgung solcher Dosen als wirksam erweisen, die unterhalb der Dosen liegen, die zur Erzielung einer - therapeutischen Zwecken genügenden - Magensäuresekretionshemmung eingesetzt werden müßten.

Verbindungen der Formel I, in denen n die Zahl 1 bedeutet, besitzen - neben ihrer Wirksamkeit gegen Helicobacter-Bakterien - auch eine ausgeprägte magensäuresekretionshemmende Wirkung. Entsprechend können diese Verbindungen auch zur Behandlung solcher Krankheiten eingesetzt werden, die auf einer erhöhten Magensäuresekretion beruhen.

Die erfindungsgemäßen Verbindungen können auch in fixer oder freier Kombination zusammen mit einer die Magensäure neutralisierenden und/oder die Magensäuresekretion hemmenden Substanz und/oder mit einer für die klassische Bekämpfung des Helicobacter pylori geeigneten Substanz verabfolgt werden.

Als die Magensäure neutralisierende Substanzen seien beispielsweise Natriumhydrogencarbonat oder andere Antacida (wie Aluminiumhydroxid, Magnesiumaluminat oder Magaldrat) genannt. Als die Magensäuresekretion hemmende Substanzen seien beispielsweise H₂-Blocker (z.B. Cimetidin, Ranitidin), H⁺/K⁺-ATPase-Hemmstoffe (z.B. Lansoprazol, Omeprazol oder insbesondere Pantoprazol) sowie sogenannte periphere Anticholinergika (z.B. Pirenzepin, Telenzepin) genannt.

Als für die klassische Bekämpfung des Helicobacter pylori geeignete Substanzen seien insbesondere antimikrobiell wirksame Substanzen wie beispielsweise Penicillin G, Gentamycin, Erythromycin, Nitrofurazon, Tinidazol, Nitrofurantoin, Furazolidon, Metronidazol und insbesondere Amoxycillin, oder aber auch Wismutsalze wie z.B. Wismutcitrat genannt.

### Biologische Untersuchungen

Die Verbindungen der Formel I wurden bezüglich ihrer Wirksamkeit gegenüber Helicobacter pylori in Anlehnung an die von Tomoyuki Iwahi et al. (Antimicrobial Agents and Chemotherapy, 1991, 490-496) beschriebene Methodik unter Verwendung von Columbia-agar (Oxoid) und bei einer Wachstumsperiode von 4 Tagen untersucht. Für die untersuchten Verbindungen ergaben sich hierbei die in der nachfolgenden Tabelle A aufgeführten ca. MIC 50-Werte (die angegebenen Nummern der Verbindungen stimmen mit den Beispielsnummern in der Beschreibung überein).

**TABELLE A**

| Verbindung Nr. | ca. MIC 50 (µg/ml) |
|---|---|
| 3 | ≤ 0,5 |
| 6 | ≤ 0,5 |
| 8 | ≤ 0,5 |
| 11 | ≤ 0,5 |
| 14 | ≤ 0,5 |
| 15 | ≤ 0,5 |
| 16 | ≤ 0,5 |
| 29 | ≤ 0,5 |
| 32 | ≤ 0,5 |

### FORMELBLATT

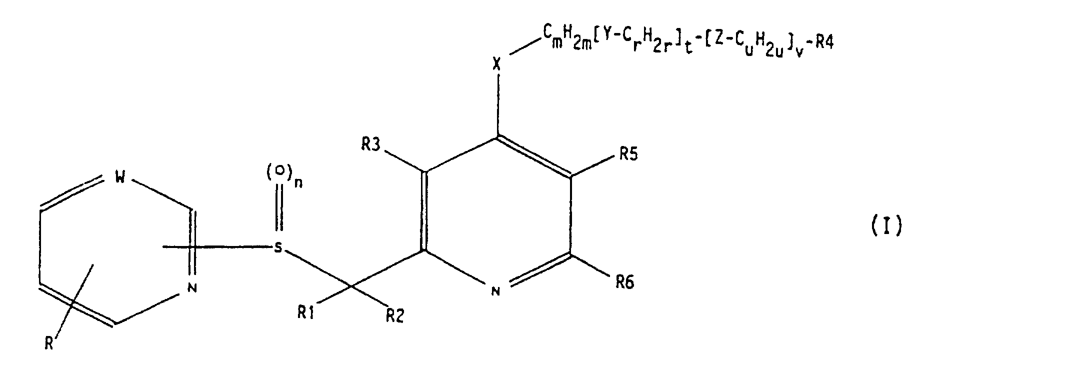

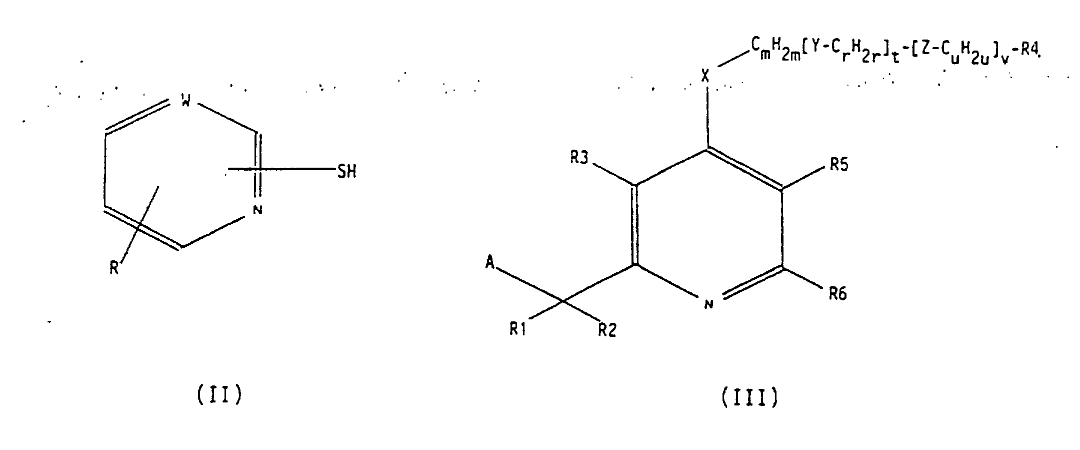

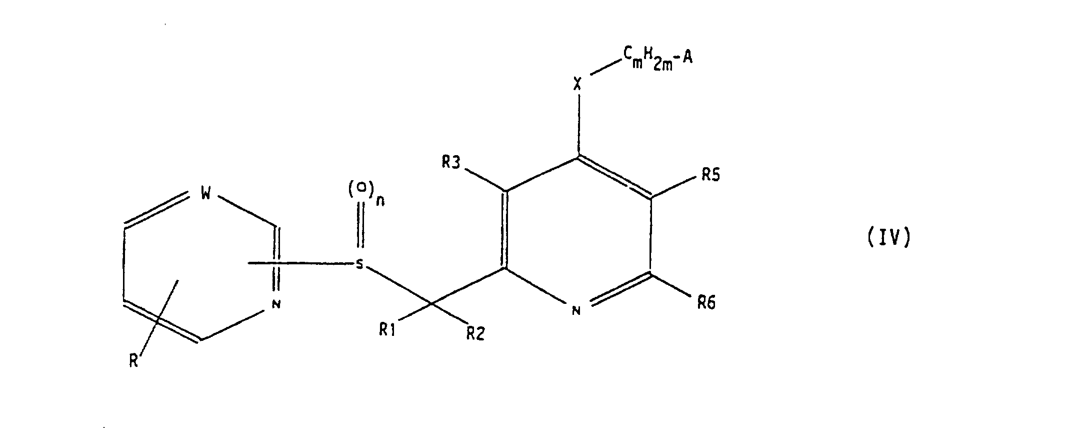

H[Y-CᵣH₂ᵣ]ₜ-[Z-CᵤH₂ᵤ]ᵥ-R4 (V)

## Patentansprüche

1. Verbindungen der Formel I, 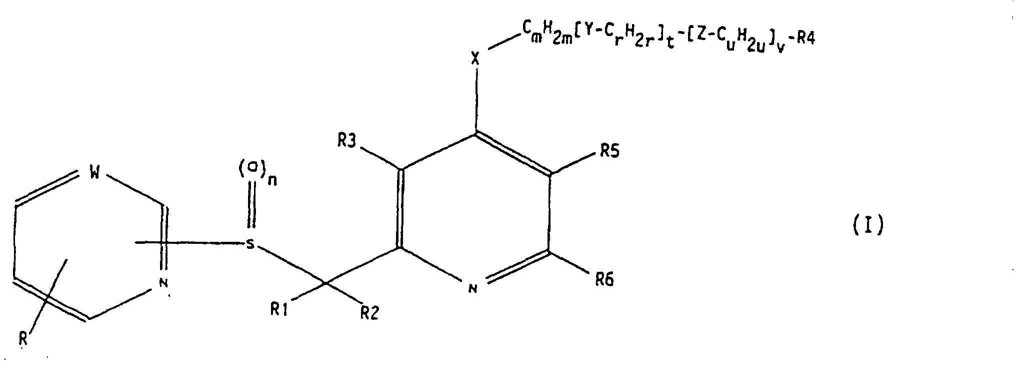 worin
R Wasserstoff, 1-4C-Alkyl, Halogen, Trifluormethyl, 1-4C-Alkoxycarbonyl, Carboxy oder Cyan bedeutet,
R1 Wasserstoff oder 1-4C-Alkyl bedeutet,
R2 Wasserstoff oder 1-4C-Alkyl bedeutet,
R3 Wasserstoff, 1-4C-Alkyl, 1-4C-Alkoxy oder Halogen bedeutet,
R4 einen Mono- oder Di-1-4C-alkylcarbamoyl- oder -thiocarbamoylrest, einen N-1-4C-Alkyl-N'-cyan-amidinorest, einen 1-N-1-4C-Alkylamino-2-nitroethylenrest, einen N-2-Propinyl-N'-cyan-amidinorest, einen Aminosulfonyl-amidinorest, den Rest -N(R7)R8 oder einen durch R9 und R10 substituierten Cyclus oder Bicyclus bedeutet, der ausgewählt ist aus der Gruppe bestehend aus Benzol, Naphthalin, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Thiadiazol-1-oxid, Oxadiazol, Pyridin, Pyridin-N-oxid, Pyrimidin, Triazin, Pyridon, Benzimidazol, Imidazopyridin, Benzthiazol, Benzoxazol und Chinolin,
R5 Wasserstoff, 1-4C-Alkyl, 1-4C-Alkoxy oder Halogen bedeutet,
R6 Wasserstoff oder 1-4C-Alkyl bedeutet,
R7 1-7C-Alkyl, 3-7C-Cycloalkyl oder Ar-1-4C-alkyl und
R8 1-7C-Alkyl, 3-7C-Cycloalkyl oder Ar-1-4C-alkyl bedeutet,
wobei
Ar Phenyl, Furyl, Naphthyl, Tetrahydronaphthyl oder durch R11, R12 und R13 substituiertes Phenyl bedeutet,
oder worin
R7 und R8 gemeinsam und unter Einschluß des Stickstoffatoms, an das beide gebunden sind, einen unsubstituierten oder substituierten 5- oder 6-Ring-Hetero(bi)cyclus darstellen, der ausgewählt ist aus der Gruppe bestehend aus Piperidin, Piperazin, Morpholin, Indolin, 1,2,3,4-Tetrahydrochinolin und 1,2,3,4-Tetrahydroisochinolin,
wobei
- ein substituierter Piperidinorest substituiert ist mit einem, zwei oder drei gleichen oder verschiedenen Substituenten ausgewählt aus der Gruppe bestehend aus 1-4C-Alkyl, 1-4C-Alkoxycarbonyl, Hydroxy-1-4C-alkyl, Phenyl, durch R11, R12 und R13 substituiertes Phenyl, Phenyl-1-4C-alkyl, Benzoyl, durch Halogen substituiertes Benzoyl und Carboxy,
- ein substituierter Piperazinorest in 2-, 3-, 5- oder 6-Position substituiert sein kann mit einem 1-4C-Alkylrest und in 4-Position substituiert ist mit einem Substituenten ausgewählt aus der Gruppe bestehend aus 1-4C-Alkyl, 3-7C-Cycloalkyl, 3-7C-Cycloalkyl-1-4C-alkyl, 1-4C-Alkoxycarbonyl-1-4C-alkyl, Carbamoyl, -CₚH₍₂ₚ₋₂₎-R14 und -C_{q}H_{2q}-R14,
- ein substituierter Morpholinorest substituiert ist mit einem oder zwei gleichen oder verschiedenen 1-4C-Alkylresten,
- ein substituierter Indolin-1-ylrest in 2- und/oder 3-Position substituiert sein kann durch eine Carboxygruppe oder durch einen oder zwei gleiche oder verschiedene 1-4C-Alkylreste, und im Benzoteil substituiert sein kann mit einem oder zwei gleichen oder verschiedenen Substituenten ausgewählt aus der Gruppe bestehend aus 1-4C-Alkyl, Halogen und Nitro,
- ein substituierter 1,2,3,4-Tetrahydrochinolinrest substituiert ist mit einem oder zwei gleichen oder verschiedenen Substituenten ausgewählt aus der Gruppe bestehend aus 1-4C-Alkyl und Halogen,
- ein substituierter 1,2,3,4-Tetrahydroisochinolinrest substituiert ist mit einem oder zwei gleichen oder verschiedenen Substituenten ausgewählt aus der Gruppe bestehend aus 1-4C-Alkyl, Carboxy und Phenyl,
R9 Wasserstoff, 1-4C-Alkyl, Hydroxy, 1-4C-Alkoxy, Halogen, Nitro, Guanidino, Carboxy, 1-4C-Alkoxycarbonyl, durch R15 substituiertes 1-4C-Alkyl oder -N(R16)R17 bedeutet,
R10 Wasserstoff, 1-4C-Alkyl, Hydroxy, 1-4C-Alkoxy, Halogen oder Trifluormethyl bedeutet,
R11 Wasserstoff, 1-4C-Alkyl, Hydroxy, 1-4C-Alkoxy, 1-4C-Alkylcarbonyl, Halogen, 1-4C-Alkylamino oder Nitro,
R12 Wasserstoff, 1-4C-Alkyl, Hydroxy, 1-4C-Alkoxy, Halogen oder Nitro, und
R13 Wasserstoff oder Trifluormethyl bedeutet,
R14 einen durch R9 und R10 substituierten Cyclus oder Bicyclus bedeutet, der ausgewählt ist aus der Gruppe bestehend aus Benzol, Naphthalin, Furan, Thiophen, Pyrrol, Oxazol, Isoxazol, Thiazol, Thiazolin, Isothiazol, Imidazol, Imidazolin, Pyrazol, Triazol, Tetrazol, Thiadiazol, Oxadiazol, Pyridin, Pyridin-N-oxid, Pyrimidin, Benzimidazol und Chinolin,
R15 Hydroxy, 1-4C-Alkoxy, Carboxy, 1-4C-Alkoxycarbonyl oder -N(R16)R17 bedeutet, wobei
R16 Wasserstoff, 1-4C-Alkyl oder -C0-R18 und
R17 Wasserstoff oder 1-4C-Alkyl bedeutet, oder wobei
R16 und R17 zusammen und unter Einschluß des Stickstoffatoms, an das beide gebunden sind, einen Piperidino- oder Morpholinorest darstellen,
R18 Wasserstoff, 1-4C-Alkyl oder 1-4C-Alkoxy bedeutet,
W CH oder N bedeutet,
X 0 (Sauerstoff), N-1-4C-Alkyl oder S bedeutet,
Y 0 (Sauerstoff), N-1-4C-Alkyl, S, SO oder SO₂ bedeutet,
Z 0 (Sauerstoff), N-1-4C-Alkyl, S, SO oder SO₂ bedeutet,
m eine Zahl von 1 bis 7 bedeutet,
n die Zahl 0, 1 oder 2 bedeutet,
r eine Zahl von 2 bis 4 bedeutet,
t die Zahl 0 oder 1 bedeutet,
u eine Zahl von 0 bis 4 bedeutet,
v die Zahl 0 oder 1 bedeutet,
p eine Zahl von 2 bis 4 bedeutet und
q eine Zahl von 0 bis 4 bedeutet
und ihre Salze,
wobei
t und/oder v nicht die Zahl 1 bedeuten, wenn m die Zahl 1 bedeutet,
Z nicht die Bedeutung SO oder SO₂ hat, wenn u die Zahl 0 bedeutet,
und wobei
R4 nicht -N(R7)R8 oder einen über N (Stickstoff) gebundenen Cyclus oder Bicyclus bedeutet, wenn Z O, S, SO oder SO₂, v die Zahl 1 und u die Zahl 0 bedeutet.

2. Verbindungen der Formel I nach Anspruch 1, worin
R Wasserstoff oder 1-4C-Alkyl bedeutet,
R1 Wasserstoff bedeutet,
R2 Wasserstoff bedeutet,
R3 Wasserstoff, 1-4C-Alkyl, 1-4C-Alkoxy oder Halogen bedeutet,
R4 einen Mono- oder Di-1-4C-alkylthiocarbamoylrest, den Rest -N(R7)R8 oder einen durch R9 und R10 substituierten Cyclus oder Bicyclus bedeutet, der ausgewählt ist aus der Gruppe bestehend aus Benzol, Furan, Thiophen, Thiazol, Isothiazol, Imidazol, Triazol, Tetrazol, Thiadiazol, Pyridin, Pyrimidin, Benzimidazol und Chinolin,
R5 Wasserstoff bedeutet,
R6 Wasserstoff oder 1-4C-Alkyl bedeutet,
R7 1-7C-Alkyl und
R8 Ar-1-4C-alkyl bedeutet,
wobei
Ar Phenyl bedeutet,
oder worin
R7 und R8 gemeinsam und unter Einschluß des Stickstoffatoms, an das beide gebunden sind, einen unsubstituierten oder substituierten 5- oder 6-Ring-Hetero(bi)cyclus darstellen, der ausgewählt ist aus der Gruppe bestehend aus Piperidin, Piperazin und 1,2,3,4-Tetrahydroisochinolin,
wobei
- ein substituierter Piperidinorest substituiert ist mit einem, zwei oder drei gleichen oder verschiedenen Substituenten ausgewählt aus der Gruppe bestehend aus 1-4C-Alkyl, Phenyl und Phenyl-1-4C-alkyl,
- ein substituierter Piperazinorest in 4-Position substituiert ist mit einem Substituenten ausgewählt aus der Gruppe bestehend aus 1-4C-Alkyl, 1-4C-Alkoxycarbonyl-1-4C-alkyl, -CₚH₍₂ₚ₋₂₎-R14 und -C_{q}H_{2q}-R14,
- ein substituierter 1,2,3,4-Tetrahydroisochinolinrest substituiert ist mit einem oder zwei gleichen oder verschiedenen Substituenten ausgewählt aus der Gruppe bestehend aus 1-4C-Alkyl und Carboxy,
R9 Wasserstoff, 1-4C-Alkyl, Halogen, Nitro, Carboxy, 1-4C-Alkoxycarbonyl oder durch R15 substituiertes 1-4C-Alkyl bedeutet,
R10 Wasserstoff oder 1-4C-Alkyl bedeutet,
R14 einen durch R9 und R10 substituierten Cyclus oder Bicyclus bedeutet, der ausgewählt ist aus der Gruppe bestehend aus Benzol, Furan, Thiophen, Thiazol, Isothiazol, Imidazol, Triazol, Tetrazol, Thiadiazol, Pyridin, Pyrimidin, Benzimidazol und Chinolin,
R15 Carboxy, 1-4C-Alkoxycarbonyl oder -N(R16)R17 bedeutet, wobei
R16 1-4C-Alkyl und
R17 1-4C-Alkyl bedeutet, oder wobei
R16 und R17 zusammen und unter Einschluß des Stickstoffatoms, an das beide gebunden sind, einen Piperidino- oder Morpholinorest darstellen,
W CH oder N bedeutet,
X S bedeutet,
Z S bedeutet,
m eine Zahl von 1 bis 5 bedeutet,
n die Zahl 0 bedeutet,
t die Zahl 0 bedeutet,
u eine Zahl von 0 bis 2 bedeutet,
v die Zahl 0 oder 1 bedeutet,
p eine Zahl von 2 bis 4 bedeutet und
q eine Zahl von 0 bis 2 bedeutet
und ihre Salze,
wobei
v nicht die Zahl 1 bedeutet, wenn m die Zahl 1 bedeutet,
und wobei
R4 nicht -N(R7)R8 oder einen über N (Stickstoff) gebundenen Cyclus oder Bicyclus bedeutet, wenn Z S bedeutet, v die Zahl 1 bedeutet und u die Zahl 0 bedeutet.

3. Verbindungen der Formel I nach Anspruch 1, worin
R Wasserstoff bedeutet,
R1 Wasserstoff bedeutet,
R2 Wasserstoff bedeutet,
R3 Wasserstoff, 1-4C-Alkyl oder 1-4C-Alkoxy bedeutet,
R4 einen Di-1-4C-alkylthiocarbamoylrest, den Rest -N(R7)R8 oder einen durch R9 und R10 substituierten Cyclus oder Bicyclus bedeutet, der ausgewählt ist aus der Gruppe bestehend aus Benzol, Furan, Thiophen, Thiazol, Imidazol, Tetrazol, Pyridin und Benzimidazol,
R5 Wasserstoff bedeutet,
R6 Wasserstoff oder 1-4C-Alkyl bedeutet,
R7 und R8 gemeinsam und unter Einschluß des Stickstoffatoms, an das beide gebunden sind, einen unsubstituierten oder substituierten Piperazinorest oder einen 1,2,3,4-Tetrahydroisochinolinrest bedeuten,
wobei
- ein substituierter Piperazinorest substituiert ist mit einem Substituenten ausgewählt aus der Gruppe bestehend aus -CₚH₍₂ₚ₋₂₎-R14 und -C_{q}H_{2q}-R14,
R9 Wasserstoff, 1-4C-Alkyl, Halogen, Nitro, Carboxy, 1-4C-Alkoxycarbonyl oder durch R15 substituiertes 1-4C-Alkyl bedeutet,
R10 Wasserstoff oder 1-4C-Alkyl bedeutet,
R14 einen durch R9 und R10 substituierten Cyclus bedeutet, der ausgewählt ist aus der Gruppe bestehend aus Benzol und Thiophen,
R15 Carboxy bedeutet,
W CH oder N bedeutet,
X S bedeutet,
Z S bedeutet,
m eine Zahl von 1 bis 3 bedeutet,
n die Zahl 0 bedeutet,
t die Zahl 0 bedeutet,
u die Zahl 0, 1 oder 2 bedeutet,
v die Zahl 0 oder 1 bedeutet,
p die Zahl 3 bedeutet und
q die Zahl 0 oder 1 bedeutet
und ihre Salze,
wobei
v nicht die Zahl 1 bedeutet, wenn m die Zahl 1 bedeutet,
und wobei
R4 nicht -N(R7)R8 oder einen über N (Stickstoff) gebundenen Cyclus oder Bicyclus bedeutet, wenn Z S bedeutet, v die Zahl 1 bedeutet und u die Zahl 0 bedeutet.

4. Verbindungen der Formel I nach Anspruch 1, worin t die Zahl 0 bedeutet und v die Zahl 0 bedeutet, und ihre Salze.

5. Verbindungen der Formel I nach Anspruch 1, worin t die Zahl 0 bedeutet, v die Zahl 1 bedeutet und u die Zahl 0 bedeutet, und ihre Salze.

6. Verbindungen der Formel I nach Anspruch 1, worin t die Zahl 0 bedeutet, v die Zahl 1 bedeutet und u die Zahl I oder 2 bedeutet, und ihre Salze.

7. Verbindungen der Formel I nach Anspruch 1, worin der Pyridin- bzw. Pyrimidinring in 2-Position gebunden ist.

8. Verbindungen der Formel I nach Anspruch 1, worin der Pyridin- bzw. Pyrimidinring in 4-Position gebunden ist.

9. Verwendung von Verbindungen der Formel I nach Anspruch 1 und ihren pharmakologisch verträglichen Salzen zur Herstellung von Arzneimitteln für die Bekämpfung von Helicobacter-Bakterien.

## Claims

1. Compounds of the formula I 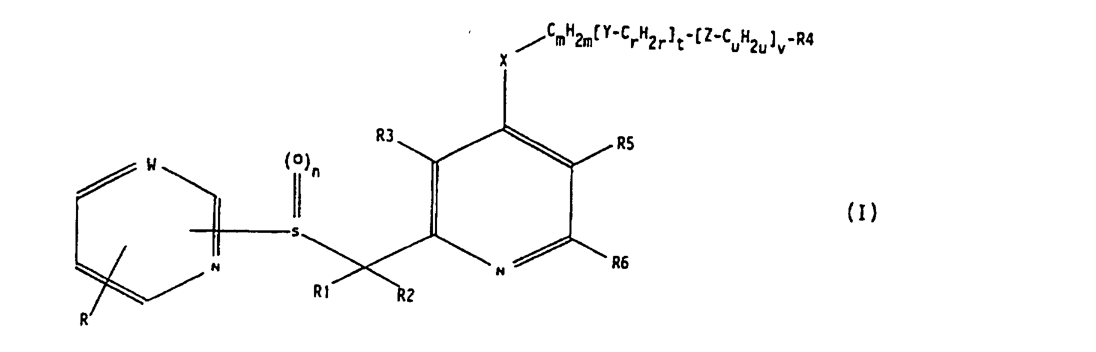 in which
R is hydrogen, 1-4C-alkyl, halogen, trifluoromethyl, 1-4C-alkoxycarbonyl, carboxyl or cyano,
R1 is hydrogen or 1-4C-alkyl,
R2 is hydrogen or 1-4C-alkyl,
R3 is hydrogen, 1-4C-alkyl, 1-4C-alkoxy or halogen,
R4 is a mono- or di-1-4C-alkylcarbamoyl or -thiocarbamoyl radical, an N-1-4C-alkyl-N'-cyanoamidino radical, a 1-N-1-4C-alkylamino-2-nitroethylene radical, an N-2-propynyl-N'-cyanoamidino radical, an aminosulfonylamidino radical, the radical -N(R7)R8 or an R9- and R10-substituted cyclic system or bicyclic system which is selected from the group consisting of benzene, naphthalene, furan, thiophene, pyrrole, oxazole, isoxazole, thiazole, thiazoline, isothiazole, imidazole, imidazoline, pyrazole, triazole, tetrazole, thiadiazole, thiadiazole-1-oxide, oxadiazole, pyridine, pyridine-N-oxide, pyrimidine, triazine, pyridone, benzimidazole, imidazopyridine, benzothiazole, benzoxazole and quinoline,
R5 is hydrogen, 1-4C-alkyl, 1-4C-alkoxy or halogen,
R6 is hydrogen or 1-4C-alkyl,
R7 is 1-7C-alkyl, 3-7C-cycloalkyl or Ar-1-4C-alkyl and
R8 is 1-7C-alkyl, 3-7C-cycloalkyl or Ar-1-4C-alkyl,
where
Ar is phenyl, furyl, naphthyl, tetrahydronaphthyl or R11-, R12- and R13-substituted phenyl,
or in which
R7 and R8 together, and including the nitrogen atom to which both are bonded, are an unsubstituted or substituted 5- or 6-membered ring hetero(bi)cyclic system, which is selected from the group consisting of piperidine, piperazine, morpholine, indoline, 1,2,3,4-tetrahydroquinoline and 1,2,3,4-tetrahydroisoquinoline,
where
- a substituted piperidino radical is substituted by one, two or three identical or different substituents selected from the group consisting of 1-4C-alkyl, 1-4C-alkoxycarbonyl, hydroxy-1-4C-alkyl, phenyl, R11-, R12- and R13-substituted phenyl, phenyl-1-4C-alkyl, benzoyl, halogen-substituted benzoyl and carboxyl,
- a substituted piperazino radical can be substituted in the 2-, 3-, 5- or 6-position by a 1-4C-alkyl radical and is substituted in the 4-position by a substituent selected from the group consisting of 1-4C-alkyl, 3-7C-cycloalkyl, 3-7C-cycloalkyl-1-4C-alkyl, 1-4C-alkoxycarbonyl-1-4C-alkyl, carbamoyl, -CₚH₍₂ₚ₋₂₎-R14 and -C_{q}H_{2q}-R14,
- a substituted morpholino radical is substituted by one or two identical or different 1-4C-alkyl radicals,
- a substituted indolin-1-yl radical can be substituted in the 2- and/or 3-position by a carboxyl group or by one or two identical or different 1-4C-alkyl radicals, and can be substituted in the benzo moiety by one or two identical or different substituents selected from the group consisting of 1-4C-alkyl, halogen and nitro,
- a substituted 1,2,3,4-tetrahydroquinoline radical is substituted by one or two identical or different substituents selected from the group consisting of 1-4C-alkyl and halogen,
- a substituted 1,2,3,4-tetrahydroisoquinoline radical is substituted by one or two identical or different substituents selected from the group consisting of 1-4C-alkyl, carboxyl and phenyl,
R9 is hydrogen, 1-4C-alkyl, hydroxyl, 1-4C-alkoxy, halogen, nitro, guanidino, carboxyl, 1-4C-alkoxycarbonyl, R15-substituted 1-4C-alkyl or -N(R16)R17,
R10 is hydrogen, 1-4C-alkyl, hydroxyl, 1-4C-alkoxy, halogen or trifluoromethyl,
R11 is hydrogen, 1-4C-alkyl, hydroxyl 1-4C-alkoxy, 1-4C-alkylcarbonyl, halogen, 1-4C-alkylamino or nitro,
R12 is hydrogen, 1-4C-alkyl, hydroxyl, 1-4C-alkoxy, halogen or nitro,
and
R13 is hydrogen or trifluoromethyl,
R14 is an R9- and R10-substituted cyclic system or bicyclic system which is selected from the group consisting of benzene, naphthalene, furan, thiophene, pyrrole, oxazole, isoxazole, thiazole, thiazoline, isothiazole, imidazole, imidazoline, pyrazole, triazole, tetrazole, thiadiazole, oxadiazole, pyridine, pyridine-N-oxide, pyrimidine, benzimidazole and quinoline,
R15 is hydroxyl, 1-4C-alkoxy, carboxyl, 1-4C-alkoxycarbonyl or -N(R16)R17, where
R16 is hydrogen, 1-4C-alkyl or -CO-R18 and
R17 is hydrogen or 1-4C-alkyl, or where
R16 and R17, together and including the nitrogen atom to which both are bonded, are a piperidino or morpholino radical,
R18 is hydrogen, 1-4C-alkyl or 1-4C-alkoxy,
W is CH or N,
X is O (oxygen), N-1-4C-alkyl or S,
Y is O (oxygen), N-1-4C-alkyl, S, SO or SO₂,
Z is O (oxygen), N-1-4C-alkyl, S, SO or SO₂,
m is a number from 1 to 7,
n is the number 0, 1 or 2,
r is a number from 2 to 4,
t is the number 0 or 1,
u is a number from 0 to 4,
v is the number 0 or 1,
p is a number from 2 to 4 and
q is a number from 0 to 4
and their salts,
where
t and/or v are not the number 1 if m is the number 1,
Z is not SO or SO₂ if u is the number 0,
and where
R4 is not -N(R7)R8 or an N (nitrogen)-bonded cyclic system or bicyclic system if Z is O, S, SO or SO₂, v is the number 1 and u is the number 0.

2. Compounds of the formula I as claimed in claim 1, in which
R is hydrogen or 1-4C-alkyl,
R1 is hydrogen,
R2 is hydrogen,
R3 is hydrogen, 1-4C-alkyl, 1-4C-alkoxy or halogen,
R4 is a mono- or di-1-4C-alkylthiocarbamoyl radical, the radical -N(R7)R8 or an R9- and R10-substituted cyclic system or bicyclic system which is selected from the group consisting of benzene, furan, thiophene, thiazole, isothiazole, imidazole, triazole, tetrazole, thiadiazole, pyridine, pyrimidine, benzimidazole and quinoline,
R5 is hydrogen
R6 is hydrogen or 1-4C-alkyl,
R7 is 1-7C-alkyl and
R8 is Ar-1-4C-alkyl,
where
Ar is phenyl,
or in which
R7 and R8, together and including the nitrogen atom to which both are bonded, are an unsubstituted or substituted 5- or 6-membered ring hetero(bi)cyclic system which is selected from the group consisting of piperidine, piperazine and 1,2,3,4-tetrahydroisoquinoline, where
- a substituted piperidino radical is substituted by one, two or three identical or different substituents selected from the group consisting of 1-4C-alkyl, phenyl and phenyl-1-4C-alkyl,
- a substituted piperazino radical is substituted in the 4-position by a substituent selected from the group consisting of 1-4C-alkyl, 1-4C-alkoxycarbonyl-1-4C-alkyl, -CₚH₍₂ₚ₋₂₎-R14 and -C_{q}H_{2q}-R14,
- a substituted 1,2,3,4-tetrahydroisoquinoline radical is substituted by one or two identical or different substituents selected from the group consisting of 1-4C-alkyl and carboxyl,
R9 is hydrogen, 1-4C-alkyl, halogen, nitro, carboxyl, 1-4C-alkoxycarbonyl or R15-substituted 1-4C-alkyl,
R10 is hydrogen or 1-4C-alkyl,
R14 is an R9- and R10-substituted cyclic system or bicyclic system which is selected from the group consisting of benzene, furan, thiophene, thiazole, isothiazole, imidazole, triazole, tetrazole, thiadiazole, pyridine, pyrimidine, benzimidazole and quinoline,
R15 is carboxyl, 1-4C-alkoxycarbonyl or -N(R16)R17, where
R16 is 1-4C-alkyl and
R17 is 1-4C-alkyl, or where
R16 and R17, together and including the nitrogen atom to which both are bonded, are a piperidino or morpholino radical,
W is CH or N,
X is S,
Z is S,
m is a number from 1 to 5,
n is the number 0,
t is the number 0,
u is a number from 0 to 2,
v is the number 0 or 1,
p is a number from 2 to 4 and
q is a number from 0 to 2
and their salts,
where
v is not the number 1 if m is the number 1,
and where
R4 is not -N(R7)R8 or an N (nitrogen)-bonded cyclic system or bicyclic system if Z is S, v is the number 1 and u is the number 0.

3. Compounds of the formula I as claimed in claim 1, in which
R is hydrogen,
R1 is hydrogen,
R2 is hydrogen,
R3 is hydrogen, 1-4C-alkyl or 1-4C-alkoxy,
R4 is a di-1-4C-alkylthiocarbamoyl radical, the radical -N(R7)R8 or an R9- and R10-substituted cyclic system or bicyclic system which is selected from the group consisting of benzene, furan, thiophene, thiazole, imidazole, tetrazole, pyridine and benzimidazole,
R5 is hydrogen,
R6 is hydrogen or 1-4C-alkyl,
R7 and R8, together and including the nitrogen atom to which both are bonded, are an unsubstituted or substituted piperazino radical or a 1,2,3,4-tetrahydroisoquinoline radical, where
- a substituted piperazino radical is substituted by a substituent selected from the group consisting of -CₚH₍₂ₚ₋₂)-R14 and -C_{q}H_{2q}-R14,
R9 is hydrogen, 1-4C-alkyl, halogen, nitro, carboxyl, 1-4C-alkoxycarbonyl or R15-substituted 1-4C-alkyl,
R10 is hydrogen or 1-4C-alkyl,
R14 is an R9- and R10-substituted cyclic system which is selected from the group consisting of benzene and thiophene,
R15 is carboxyl,
W is CH or N,
X is S,
Z is S,
m is a number from 1 to 3,
n is the number 0,
t is the number 0,
u is the number 0, 1 or 2,
v is the number 0 or 1,
p is the number 3 and
q is the number 0 or 1
and their salts,
where
v is not the number 1 if m is the number 1,
and where
R4 is not -N(R7)R8 or an N (nitrogen)-bonded cyclic system or bicydic system if Z is S, v is the number 1 and u is the number 0.

4. Compounds of the formula I as claimed in claim 1, in which t is the number 0 and v is the number 0, and their salts.

5. Compounds of the formula I as claimed in claim 1, in which t is the number 0, v is the number 1 and u is the number 0, and their salts.

6. Compounds of the formula I as claimed in claim 1, in which t is the number 0, v is the number 1 and u is the number 1 or 2, and their salts.

7. Compounds of the formula I as claimed in claim 1, in which the pyridine or pyrimidine ring is bonded in the 2-position.

8. Compounds of the formula I as claimed in claim 1, in which the pyridine or pyrimidine ring is bonded in the 4-position.

9. The use of compounds of the formula I as claimed in claim 1 and their pharmacologically tolerable salts for the production of medicaments for the control of Helicobacter bacteria.

## Revendications

1. Composés de formule (I) 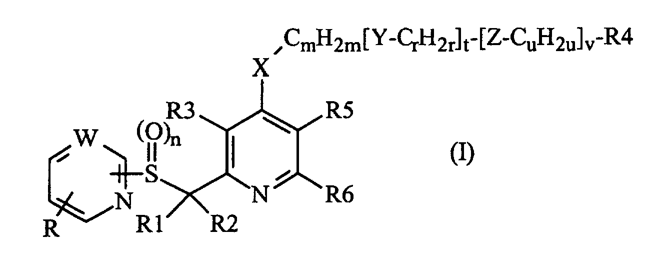 dans laquelle
R représente un atome d'hydrogène, un groupe alkyle en C₁₋₄, un atome d'halogène, un groupe trifluorométhyle, (alcoxy en C₁₋₄)-carbonyle, carboxyle ou cyano,
R1 représente un atome d'hydrogène ou un groupe alkyle en C₁₋₄,
R2 représente un atome d'hydrogène ou un groupe alkyle en C₁₋₄,
R3 représente un atome d'hydrogène, un groupe alkyle en C₁₋₄, alcoxy en C₁₋₄ ou halogéno,
R4 représente un groupe mono- ou di-(alkyle en C₁₋₄)-carbamoyle ou - thiocarbamoyle, N-(alkyle en C₁₋₄)-N'-cyano-amidino, 1-N-(alkyle en C₁₋₄)-amino-2-nitroéthylène, N-2-propinyl-N'-cyano-amidino, aminosulfonylamidino, -N(R7)R8 ou un résidu monocyclique ou bicyclique choisi parmi benzène, naphtalène, furane, thiophène, pyrrole, oxazole, isoxazole, thiazole, thazoline, isothiazole, imidazole, imadazoline, pyrazole, triazole, tétrazole, thiadiazole, thiadiazole-1-oxyde, oxadiazole, pyridine, pyridine-N-oxyde, pyrimidine, triazine, pyridone, benzimidazole, imidazopyridine, benzothiazole, benzoxazole et quinoline, portant des substituants R9 et R10,
R5 représente un atome d'hydrogène, un groupe alkyle en C₁₋₄, alcoxy en C₁₋₄ ou halogéno,
R6 représente un atome d'hydrogène ou un groupe alkyle en C₁₋₄,
R7 représente un groupe alkyle en C₁₋₇, cycloalkyle en C₃₋₇ ou Ar(alkyle en C₁₋₄), et
R8 représente un groupe alkyle en C₁₋₇, cycloalkyle en C₃₋₇ ou Ar(alkyle en C₁₋₄),
où
Ar représente un groupe phényle, furyle, naphtyle, tétrahydronaphtyle ou phényle portant des substituants R11, R12 et R13, ou
R7 et R8 représentent ensemble et avec l'atome d'azote auquel ils sont liés, un hétéro(bi)cycle à 5 ou 6 chaînons, substitué ou non, choisi dans le groupe formé par pipéridine, pipérazine, morpholine, indoline, 1,2,3,4-tétrahydroquinoline et 1,2,3,4-tétrahydroisoquinoline,
où
- un résidu pipéridino substitué porte un, deux ou trois substituants identiques ou différents, choisis parmi alkyle en C₁₋₄, (alcoxy en C₁₋₄)-carbonyle, hydroxy(alkyle en C₁₋₄), phényle, phényle portant des substituants R11, R12 et R13, phényl(alkyle en C₁₋₄), benzoyle, benzoyle halogéné et carboxyle,
- un résidu pipérazino substitué peut porter en position 2, 3, 5 ou 6 un substituant alkyle en C₁₋₄ et en position 4 un substituant choisi parmi alkyle en C₁₋₄, cycloalkyle en C₃₋₇, (cycloalkyle en C₃₋₇)-(alkyle en C₁₋₄), (alcoxy en C₁₋₄)-carbonyl-(alkyle en C₁₋₄), carbamoyle, -CₚH₍₂ₚ₋₂₎-R14 et -C_{q}H_{2q}-R14,
- un résidu morpholino substitué porte un ou deux substituants alkyle en C₁₋₄, identiques ou différents,
- un résidu indolin-1-yle substitué peut porter en position 2 et/ou 3 un substituant carboxyle ou un ou deux substituants alkyle en C₁₋₄, identiques ou différents, et peut porter sur le cycle benzo un ou deux substituants identiques ou différents, choisis parmi alkyle en C₁₋₄, halogéno et nitro,
- un résidu 1,2,3,4-tétrahydroquinoline substitué porte un ou deux substituants, identiques ou différents, choisis parmi alkyle en C₁₋₄ et halogéno,
- un résidu 1,2,3,4-tétrahydroisoquinolino substitué porte un ou deux substituants, identiques ou différents, choisis parmi alkyle en C₁₋₄, carboxyle et phényle,
R9 représente un atome d'hydrogène, un groupe alkyle en C₁₋₄, hydroxyle, alcoxy en C₁₋₄, halogéno, nitro, guanidino, carboxyle, (alcoxy en C₁₋₄)-carbonyle, alkyle en C₁₋₄ portant un substituant R15 ou -N(R16)R17,
R10 représente un atome d'hydrogène, un groupe alkyle en C₁₋₄, hydroxyle, alcoxy en C₁₋₄, halogéno ou trifluorométhyle,
R11 représente un atome d'hydrogène, un groupe alkyle en C₁₋₄, hydroxyle, alcoxy en C₁₋₄, (alkyle en C₁₋₄)-carbonyle, halogéno, (alkyle en C₁₋₄)-amino ou nitro,
R12 représente un atome d'hydrogène, un groupe alkyle en C₁₋₄, hydroxyle, alcoxy en C₁₋₄, halogéno ou nitro, et
R13 représente un atome d'hydrogène ou un groupe trifluorométhyle,
R14 représente un résidu mono- ou bicyclique choisi parmi benzène, naphtalène, furane, thiophène, pyrrole, oxazole, isoxazole, thiazole, thiazoline, isothiazole, imidazole, imadazoline, pyrazole, triazole, tétrazole, thiadiazole, oxadiazole, pyridine, pyridine-N-oxyde, pyrimidine, benzimidazole et quinoline, portant des substituants R9 et R10,
R15 représente un groupe hydroxy, alcoxy en C₁₋₄, carboxy, (alcoxy en C₁₋₄)-carbonyle ou -N(R16)R17), avec
R16 représentant un atome d'hydrogène ou un groupe alkyle en C₁₋₄ ou -CO-R18, et
R17 représente un atome d'hydrogène ou un groupe alkyle en C₁₋₄, ou
R16 et R17 représentent ensemble, avec l'atome d'azote auquel ils sont liés, un résidu pipéridino ou morpholino,
R18 représente un atome d'hydrogène, un groupe alkyle en C₁₋₄ ou alcoxy en C₁₋₄,
W représente un groupe CH ou N,
X représente un atome d'oxygène (O), un groupe N-(alkyle en C₁₋₄) ou un atome de soufre (S),
Y représente un atome d'oxygène (O), un groupe N-(alkyle en C₁₋₄), un atome de soufre (S), un groupe SO ou SO₂,
Z représente un atome d'oxygène (O), un groupe N-(alkyle en C₁₋₄), un atome de soufre (S), un groupe SO ou SO₂,
m vaut de 1 à 7,
n vaut 0, 1 ou 2,
r vaut de 2 à 4,
t vaut 0 ou 1,
u vaut de 0 à 4,
v vaut 0 ou 1,
p vaut de 2 à 4, et
q vaut de 0 à 4
et les sels de tels composés,
avec la réserve que
t et/ou v ne valent pas 1 lorsque m vaut 1,
Z ne signifie pas SO ou SO₂ lorsque u vaut 0, et
R4 ne représente pas un résidu -N(R7)(R8) ou un cycle ou bicycle lié par l'intermédiaire d'un atome d'azote, lorsque Z est O, S, SO ou SO₂, v vaut 1 et u vaut 0.

2. Composés de formule I selon la revendication 1 dans laquelle R représente un atome d'hydrogène ou un groupe alkyle en C₁₋₄,
R1 représente un atome d'hydrogène,
R2 représente un atome d'hydrogène,
R3 représente un atome d'hydrogène, un groupe alkyle en C₁₋₄, alcoxy en C₁₋₄ ou halogéno,
R4 représente un groupe mono- ou di-(alkyle en C₁₋₄)-thiocarbamoyle, -N(R7)R8 ou un résidu monocyclique ou bicyclique choisi parmi benzène, furane, thiophène, thiazole, isothiazole, imidazole, triazole, tétrazole, thiadiazole, pyridine, pyrimidine, benzimidazole et quinoline, portant des substituants R9 et R10,
R5 représente un atome d'hydrogène,
R6 représente un atome d'hydrogène ou un groupe alkyle en C₁₋₄,
R7 représente un groupe alkyle en C₁₋₇, et
R8 représente un groupe Ar(alkyle en C₁₋₄),
où
Ar représente un groupe phényle, ou
R7 et R8 représentent ensemble et avec l'atome d'azote auquel ils sont liés, un hétéro(bi)cycle à 5 ou 6 chaînons, substitué ou non, choisi dans le groupe formé par pipéridine, pipérazine et 1,2,3,4-tétrahydroisoquinoline,
où
- un résidu pipéridino substitué porte un, deux ou trois substituants, identiques ou différents, choisis parmi alkyle en C₁₋₄, phényle et phényl(alkyle en C₁₋₄),
- un résidu pipérazino substitué porte en position 4 un substituant choisi parmi alkyle en C₁₋₄, (alcoxy en C₁₋₄)-carbonyl-(alkyle en C₁₋₄), -CₚH₍₂ₚ₋₂₎-R14 et -C_{q}H_{2q}-R14,
- un résidu 1,2,3,4-tétrahydroisoquinolino substitué porte un ou deux substituants, identiques ou différents, choisis parmi alkyle en C₁₋₄ et carboxyle,
R9 représente un atome d'hydrogène, un groupe alkyle en C₁₋₄, halogéno, nitro, carboxyle, (alcoxy en C₁₋₄)-carbonyle ou alkyle en C₁₋₄ portant un substituant R15,
R10 représente un atome d'hydrogène ou un groupe alkyle en C₁₋₄,
R14 représente un résidu mono- ou bicyclique choisi parmi benzène, furane, thiophène, thiazole, isothiazole, imidazole, triazole, tétrazole, thiadiazole, pyridine, pyrimidine, benzimidazole et quinoline, portant des substituants R9 et R10,
R15 représente un groupe carboxy, (alcoxy en C₁₋₄)-carbonyle ou - N(R16)R17), avec
R16 représentant un groupe alkyle en C₁₋₄, et
R17 représente un groupe alkyle en C₁₋₄, ou
R16 et R17 représentent ensemble, avec l'atome d'azote auquel ils sont liés, un résidu pipéridino ou morpholino,
W représente un groupe CH ou N,
X représente un atome de soufre (S),
Z représente un atome de soufre (S),
m vaut de 1 à 5,
n vaut 0,
t vaut 0,
u vaut de 0 à 2,
v vaut 0 ou 1,
p vaut de 2 à 4, et
q vaut de 0 à 2,
et les sels de tels composés,
avec la réserve que
v ne vaut pas 1 lorsque m vaut 1, et
R4 ne représente pas un résidu -N(R7)R8 ou un monocycle ou bicycle lié par l'intermédiaire d'un atome d'azote, lorsque Z est S, v vaut 1 et u vaut 0.

3. Composés de formule I selon la revendication 1 dans laquelle R représente un atome d'hydrogène,
R1 représente un atome d'hydrogène,
R2 représente un atome d'hydrogène,
R3 représente un atome d'hydrogène, un groupe alkyle en C₁₋₄ ou alcoxy en C₁₋₄,
R4 représente un groupe di-(alkyle en C₁₋₄)-thiocarbamoyle, -N(R7)R8 ou un résidu monocyclique ou bicyclique choisi parmi benzène, furane, thiophène, thiazole, imidazole, tétrazole, pyridine et benzimidazole, portant des substituants R9 et R10,
R5 représente un atome d'hydrogène,
R6 représente un atome d'hydrogène ou un groupe alkyle en C₁₋₄,
R7 et R8 représentent ensemble et avec l'atome d'azote auquel ils sont liés, un résidu pipérazino ou 1,2,3,4-tétrahydroisoquinoline, substitué ou non,
- un résidu pipérazino substitué étant un résidu portant un substituant choisi parmi -CₚH₍₂ₚ₋₂₎-R14 et -C_{q}H_{2q}-R14,
R9 représente un atome d'hydrogène, un groupe alkyle en C₁₋₄, halogéno, nitro, carboxyle, (alcoxy en C₁₋₄)-carbonyle ou alkyle en C₁₋₄ portant un substituant R15,
R10 représente un atome d'hydrogène ou un groupe alkyle en C₁₋₄,
R14 représente un résidu benzène ou thiophène, portant des substituants R9 et R10,
R15 représente un groupe carboxyle,
W représente un groupe CH ou N,
X représente un atome de soufre (S),
Z représente un atome de soufre (S),
m vaut de 1 à 3,
n vaut 0,
t vaut 0,
u vaut 0, 1 ou 2,
v vaut 0 ou 1,
p vaut 3, et
q vaut 0 ou 1,
et les sels de tels composés,
avec la réserve que
v ne vaut pas 1 lorsque m vaut 1, et
R4 ne représente pas un résidu -N(R7)(R8) ou un monocycle ou bicycle lié par l'intermédiaire d'un atome d'azote, lorsque Z est S, v vaut 1 et u vaut 0.

4. Composés de formule (I) selon la revendication 1 où t vaut 0 et v vaut 0, et les sels de ces composés.

5. Composés de formule (I) selon la revendication 1, dans lesquels t vaut 0, v vaut 1 et u vaut 0, et les sels de ces composés.

6. Composés de formule (I) selon la revendication 1, dans lesquels t vaut 0, v vaut 1 et u vaut 1 ou 2, et les sels de ces composés.

7. Composés de formule (I) selon la revendication 1, dans lesquels le cycle pyridine ou pyrimidine est lié en position 2.

8. Composés de formule (I) selon la revendication 1, dans lesquels le cycle pyridine ou pyrimidine est lié en position 4.

9. Utilisation de composés de formule (I) selon la revendication 1 et de leurs sels pharmacologiquement acceptables pour la préparation de médicaments destinés à lutter contre des bactéries *Helicobacter*.
